(19) Europäisches Patentamt
European Patent Office
Office européen des brevets

(11) EP 1 358 238 B1

(12) **FASCICULE DE BREVET EUROPEEN**

(45) Date de publication et mention de la délivrance du brevet:
**07.04.2010 Bulletin 2010/14**

(21) Numéro de dépôt: **01989673.7**

(22) Date de dépôt: **28.12.2001**

(51) Int Cl.:
***C08G 18/02*** *(2006.01)* ***C08G 18/09*** *(2006.01)*
***C08G 18/24*** *(2006.01)* ***C07D 229/00*** *(2006.01)*

(86) Numéro de dépôt international:
**PCT/FR2001/004228**

(87) Numéro de publication internationale:
**WO 2002/053614 (11.07.2002 Gazette 2002/28)**

(54) **CATALYSEUR DE CONDENSATION POUR ISOCYANATE, COMPOSITION EN CONTENANT, PROCEDE D'UTILISATION, ET COMPOSITIONS OBTENUES**

KATALYSATOR ZUR ISOCYANATKONDENSATION, ZUSAMMENSETZUNG, DIE DIESEN ENTHÄLT, VERFAHREN ZUR VERWENDUNG UND ERHALTENE ZUSAMMENSETZUNGEN

CATALYST FOR ISOCYANATE CONDENSATION, COMPOSITION CONTAINING SAME, METHOD FOR USE, AND RESULTING COMPOSITIONS

(84) Etats contractants désignés:
**AT BE CH CY DE DK ES FI FR GB GR IE IT LI LU MC NL PT SE TR**

(30) Priorité: **29.12.2000 FR 0017315**
**29.12.2000 FR 0017317**
**29.12.2000 FR 0017323**
**21.12.2001 FR 0116723**

(43) Date de publication de la demande:
**05.11.2003 Bulletin 2003/45**

(73) Titulaire: **Perstorp France**
**69800 Saint-Priest (FR)**

(72) Inventeurs:
- **BERNARD, Jean-Marie**
**Saint-Laurent D'Agny,**
**F-69440 Mornant (FR)**
- **BONNEAU, Damien**
**F-89100 Saint Clement (FR)**
- **VISSEAU, Marc**
**F-21240 Talant (FR)**

(74) Mandataire: **Colombet, Alain André et al**
**Cabinet Lavoix**
**2, Place d'Estienne d'Orves**
**75441 Paris Cedex 09 (FR)**

(56) Documents cités:
**US-A- 4 003 859** **US-A- 4 675 401**
**US-A- 5 017 695**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 358 238 B1

## Description

L'invention concerne un nouveau procédé de (cyclo)condensation, notamment de (cyclo)trimérisation catalytique d'isocyanates.

La réaction de trimérisation notamment de cyclotrimérisation des isocyanates est une réaction connue catalysée généralement par :

- des catalyseurs basiques : ammoniums quaternaires ou leurs dérivés hydroxyalcoyles, alcoolates, phénolates, bases de Mannich, amines tertiaires, sels d'imides, ...
- des catalyseurs organométalliques : titanates d'alcoyle, carboxylates de plomb, ...

Toutefois, la cinétique de cette réaction est dépendante de la structure de l'isocyanate et il peut être opportun de choisir le catalyseur en fonction de la nature de l'isocyanate.

Généralement, les carboxylates d'ammoniums quaternaires à fonction hydroxyalcoyle, sont les catalyseurs de choix pour les diisocyanates aliphatiques. Ils donnent de bons résultats à basse température (70 à 80°C) et en faible quantité (de l'ordre de 0.07% en poids par rapport au diisocyanate).

Le brevet européen EP 57653 (Rhône Poulenc) décrit l'hexaméthyldisilazane (HMDZ) comme catalyseur de trimérisation des diisocyanates aliphatiques. Toutefois, les quantités de catalyseur utilisées et les températures de réaction sont nettement supérieurs à celles mises en oeuvre pour les carboxylates d'ammonium quaternaire, par exemple de l'ordre de 1,25% en poids de catalyseur par rapport à l'HDI et 120°C.

Les travaux des inventeurs ayant conduit à la présente invention ont permis de découvrir que la réaction de polycondensation, notamment de trimérisation d'isocyanates monomères pouvait être effectuée avec des sels à structures de silazanes ou de silanolates, (composés non toxiques) mis en oeuvre en faible quantité à une température peu élevée, avec une excellente productivité et ce sans entraîner de coloration notable du milieu réactionnel.

De manière surprenante, la cinétique de la réaction de trimérisation ainsi que la réactivité des isocyanates monomères sont substantiellement améliorées lorsque l'on utilise un sel de silazane par rapport au dérivé de silazane protoné correspondant.

On a, en outre, découvert de manière surprenante que les sels de silazane ou les silanolates permettaient de catalyser la polycondensation, notamment la (cyclo)trimérisation d'isocyanates dans lesquels la fonction isocyanate est encombrée donc peu accessible, notamment les isocyanates dans lesquels la fonction isocyanate est portée par un atome de carbone secondaire, notamment cycloaliphatique, tertiaire ou néopentylique. Qui plus est, on peut jouer sur les cations associés pour pondérer d'une manière inusuelle les différentes espèces d'oligocondensats issus de la (cyclo) condensation.

Ainsi selon l'invention on utilise des composés salins comme catalyseurs de (cyclo)condensation de fonction isocyanate avantageusement aliphatique, y compris cycloaliphatique, dans laquelle ledit composé salin est choisi parmi ceux qui répondent à la formule:

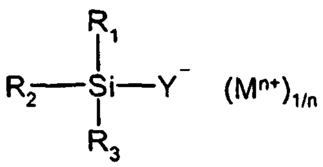

où $(M^{n+})_{1/n}$ symbolise le(s) co-cation(s) nécessaire(s) à la neutralité électrique dudit composé salin équilibrant l'anion de formule (I) :

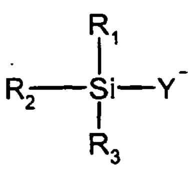
(I)

où $Y^-$ est choisi parmi :

- un oxygène chargé négativement ;

- et un radical carboné comportant une charge négative portée par un atome de la colonne VB ou par un oxygène, avantageusement par un azote et dont la liaison avec le silicium de la formule (I) ci-dessus est portée par un atome de la colonne VB, avantageusement par un azote ;

où $R_1$, $R_2$ et $R_3$, identiques ou différents, représentent un groupe monovalent hydrocarboné ayant avantageusement de 1 à 30 atomes de carbone, éventuellement substitué par un ou plusieurs atomes d'halogène, groupements CN, ou ester, et/ou éventuellement interrompu par un ou plusieurs atomes choisis parmi O, N et Si, avantageusement de nature aliphatique, y compris cycloaliphatique saturé ou insaturé et aralcoyle, ou de nature aromatique tel que aryle ou alcoylaryle ;
et parmi ceux porteurs d'un groupe trialcoylsilyle et résultant de la réaction desdits composés de formule (I) et d'une fonction isocyanate avantageusement aliphatique.

En effet, les composés anioniques de formule (I) sont susceptibles de former des composés avec les isocyanates, notamment issu de l'addition sur la fonction isocyanate. Les additions de l'anion se font sur le carbone de la fonction isocyanate.

Avantageusement, Y représente un radical de formule (II) :

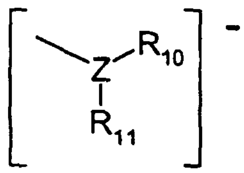

(II)

dans laquelle :

- Z représente un métalloïde de la colonne VB ;
- $R_{10}$ est choisi parmi les radicaux comportant de 1 à 30 atomes de carbone, y compris silyle et notamment trialcoylsilyle ; et
- $R_{11}$ est choisi parmi une charge négative (anionique) et un groupe carboné (avantageusement d'au plus 30 atomes de carbone) attaché à Z par un carbone à la fois d'hybridation $_{sp}{}^2$ et porteur d'un azote dont l'azote porte une charge négative (anionique) ;

Selon une réalisation de l'invention, il est possible mais sans que cela présente d'avantage particulier que le composé anionique de formule 1 présente plus d'une séquence catalytiquement active ; selon cette possibilité, dans ce cas l'un au moins des $R_1$, $R_2$, et $R_3$, est interrompu par plusieurs atomes choisis parmi O, N et Si, formant la séquence (I') :

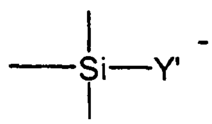

dans laquelle $Y'^-$ représente un atome d'oxygène chargé négativement ou une séquence (II') :

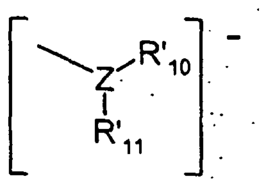

dans laquelle

- Z' possède les mêmes valeurs que Z ;
- $R'_{10}$, représente un groupe hydrocarboné relié au radical Z' par un carbone ou un silicium ; (cf. radical $R_{10}$)
- $R'_{11}$ étant la charge négative de Y' ou une séquence sur un atome d'azote de Y' comportant un carbone $sp^2$ à la

fois lié à Z' et porteur d'un azote porteur de la charge négative.

Selon un mode préféré de la présente invention, le composé anionique de formule (I) quand $R_{11}$ est une charge négative, $R_{10}$ présente avantageusement au plus 30 atomes de carbone et est choisi parmi les radicaux hydrocarbonés, notamment les aryles, les alcoyles et les silyles et parmi les groupes carbonés attachés à Z par un carbone, lequel est à la fois d'hybridation $sp^2$ et porteur d'un azote.

Dans ce dernier cas, il est préféré que quand $R_{11}$ est une charge négative, $R_{10}$ présente avantageusement au plus 30 atomes de carbone et est choisi parmi les radicaux alcoyles, silyles, et les groupes carbonés attachés à Z par le carbone d'hybridation $sp^2$ une séquence iminométhylènyle

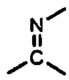

ou d'une séquence -C(O)-N<.

De préférence $R_{10}$ est alors de formule :

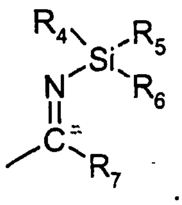

Lorsque $R_{10}$ est de formule (III) :

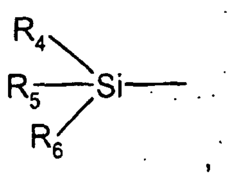

il est préférable que $R_{11}$ représente alors une charge négative ou soit de formule (IV) :

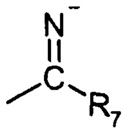

dans laquelle :

- $R_4$, $R_5$ et $R_6$ identiques ou différents représentent un groupe monovalent de nature hydrocarbonée ayant de 1 à 30 atomes de carbone, aliphatique, cycloaliphatique saturé ou insaturé, aryle, aralcoyle ou alcoylaryle, éventuellement substitué par un ou plusieurs atomes d'halogène, groupements CN, ou ester, et/ou éventuellement interrompu par un ou plusieurs atomes choisis parmi O, S, N et Si ;
- $R_7$ représente avantageusement un radical hydrocarboné d'au plus 30 carbones, avantageusement de nature aliphatique (c'est-à-dire dont lé carbone porteur du lien avec l'imine est d'hybridation $sp^3$) notamment alcoyle, ou avantageusement un aryle de préférence isocyclique.

Ainsi, selon un mode préféré de la présente invention le composé (I) répond à l'une des formules (Va) ou (Vb) suivantes :

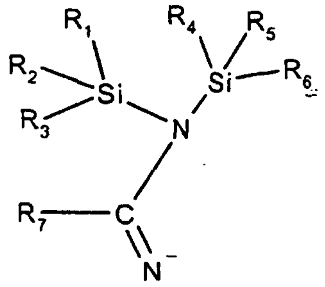

;

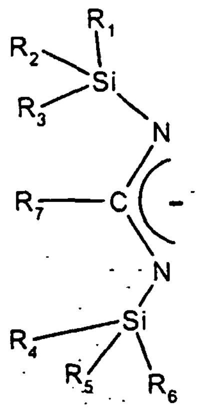

(Va) (Vb)

Ces composés peuvent être obtenus par l'action de l'anion de formule (1) sur un nitrile ($R_7$-CN). On obtient alors le composé de formule (Va) qui s'isomérise en composé de formule (Vb).

Selon un autre mode avantageux de réalisation, ledit composé de formule (I) est choisi parmi les composés de formules (1 ) et (2) :

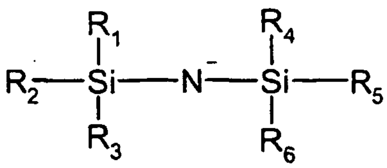

(1)

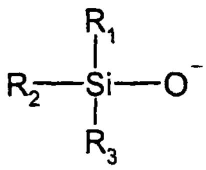

(2)

où $R_1$, $R_2$, $R_3$, $R_4$, $R_5$ et $R_6$, identiques ou différents, représentent un groupe monovalent de nature hydrocarbonée ayant de 1 à 30 atomes de carbone, aliphatique, cycloaliphatique saturé ou insaturé, aryle, aralcoyle ou alcoylaryle, éventuellement substitué par un ou plusieurs atomes d'halogène, groupements CN, ou ester, et/ou éventuellement interrompu par un ou plusieurs atomes choisis parmi O, S, N et Si.

Tous les co-cations non-porteurs d'hydrogène acide (c'est-à-dire dont le pKa est inférieur à 20, et mieux à 30) donnent des résultats satisfaisants. Pour éviter des réactions parasites d'oxydo-réduction, il est préférable, mais non indispensable, que les cations correspondent à des éléments qui hors l'état élémentaire ne possèdent qu'un seul état oxydation. Quand ils en possèdent plusieurs, il est préférable qu'ils soient sous l'état oxydé le moins valent.

Usuellement, on préfère toutefois parmi lesdits cations minéraux, les alcalins de rang au moins égal à celui du sodium. On peut également utiliser des cations organiques ayant des propriétés équivalentes, comme par exemple les fluorures d'oniums (c'est à dire les cations dont le nom est formé par l'addition du suffixe onium, tel que sulfonium, phosphonium, ammonium,...), du type tétra-alcoylammonium tétra-arylphosphonium, trialcoylsulfonium ; ou plus rarement des iniums (c'est à dire les cations dont le nom est formé par l'addition du suffixe inium, tel que sulfinium, phosphinium, iminium,...), tels les pyridiniums.

Ces oniums, sont avantageusement dans les cations formés par les éléments métalloïdes des colonnes VB et VIB (tels que définis dans le tableau de la classification périodique des éléments publiés au supplément au Bulletin de la Société Chimique de France en janvier 1966) avec respectivement 4 ou 3 chaînes hydrocarbonées monovalentes. Il convient de noter que les oxoniums sont peu stables et ne peuvent être utilisés dans cette application.

Les iniums formés par les éléments métalloïdes des colonnes VB et VIB (tels que définis dans le tableau de la classification périodique des éléments publiés au supplément au Bulletin de la Société Chimique de France en janvier

1966) avec respectivement 3 ou 2 chaînes hydrocarbonées dont une est bivalente et forme une double liaison avec ledit élément métalloïde et les autres monovalentes.

Parmi ces oniums, les préférés sont les tétra-alcoylammoniums de 4 à 24 atomes de carbone, de préférence de 4 à 12 atomes de carbone. Le tétra-alcoylammonium est en général du tétraméthylammonium. Et les tétra-alcoylphosphoniums [dans la présente description ALCO-*yle* est pris dans son sens étymologique de reste hydrocarboné d'un ALca-*ol* après ignorance de la fonction alcool (ou ol)].

Le choix des desdits co-cations peut jouer un rôle sur la répartition des différents (cyclo)condensats.

Lorsqu'ils sont choisis parmi les cations favorisant le caractère dissocié tels que iniums, et surtout oniums et parmi les cations métalliques alcalins, la répartition entre les différentes espèces n'est pas très différente de celle obtenue par l'HMDZ. Le pouvoir catalytique en revanche est multiplié par un facteur souvent supérieur à 10.

On peut également utiliser les cations polyvalents tels que les cations des composés alcalino-terreux et des terres rares, et surtout des métaux de la colonne de l'aluminium ou du silicium, et des métaux de transition, ces métaux permettent une meilleure maîtrise de la cinétique.

Mention particulière des cations des terres rares y compris cations du lanthane et des lanthanides et notamment de l'erbium doit être faite. L'erbium, en particulier comme cation des catalyseurs de formule (1) tel que définis précédemment, donne des compositions plus riches en un composé qui n'est présent qu'à l'état de traces difficilement mesurables, lorsque l'on utilise des techniques de trimérisation de l'état antérieur de la technique. Il s'agit également de composés trimères, mais ayant un cycle 6-amino-5-azauracile de formule :

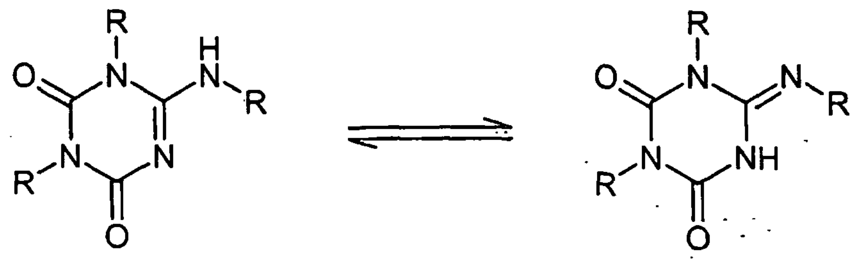

Dans ce cas spécifique, la teneur en ce composé dépend majoritairement de la quantité de catalyseur utilisée. La basicité de ces produits leur confère un intérêt particulier, seuls, ou de préférence dans les compositions isocyanates usuelles telles que celles connues sous les appellations de trimère ou de biuret.

Les R, identiques ou différents, représentent les restes du ou des monomères, après ignorance d'une des fonctions isocyanates, les autres pouvant être engagées dans une condensation distincte.

L'utilisation de sels d'étain, et notamment d'étain (II) comme cation des composés de formule (I), conduit à la formation majoritaire de IUT (2-imino-4-oxo-1,3-diazétidine) de formule :

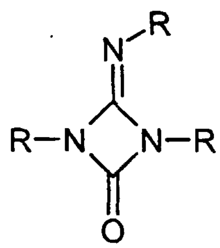

où les R, identiques ou différents, représentent les restes du, ou des monomères, après ignorance d'une des fonctions isocyanates, les autres pouvant être engagées dans une condensation distincte.

La basicité de ces deux types de produits ci-dessus leur confère un intérêt particulier, seuls, ou de préférence dans les compositions prépolymères isocyanates usuelles, telles que celles connues sous les appellations de trimère ou de biuret, et ce, à hauteur d'au moins 1% en masse, avantageusement d'au moins 3%, de préférence au mois 5%. Elles sont utiles, en particulier _pour les revêtements les colles et les micro-encapsulations.

Comme compositions prépolymères isocyanates et comportant en mélange soit les composés à motif 6-amino-5-azauracile soit à motif 2-imino-4-oxo-1,3-diazétidine, parfois désignée par le sigle IUT (IminoUreTidinedione) sont avantageusement choisies parmi celles obtenues par une oligocondensation de monomères diisocyanates voire de monomères triisocyanates (de préférence diisocyanates) et élimination éventuelle des monomères résiduels ou par

mélange de deux ou plusieurs sous-compositions du type ci-dessus et issues d'oligocondensation réalisées dans des conditions (température, catalyseurs) distinctes.

Il est souhaitable que ces compositions présentent une fonctionnalité moyenne avantageusement supérieure à 2,5, de préférence supérieure à 3, de préférence et avantageusement au plus égale à 8 et de préférence à 6, plus préférentiellement à 4, comportant :

(a) de 0 à 20%, avantageusement au moins 0,5% en masse par rapport à la masse totale des composants a), b) et c), de composés porteurs d'une seule fonction uretidinedione ; et dont la masse moléculaire est au plus égale à trois fois la masse moléculaire du monomère (ou du monomère le plus lourd s'il y a plusieurs monomères) desdits monomères isocyanates ;
(b) de 0 à 65%, avantageusement au moins 10% en masse par rapport à la masse totale des composants a), b) et c), de composés porteurs soit d'un seul motif isocyanurate soit d'un seul motif biuret et dont la masse moléculaire est au plus égale à cinq fois la masse moléculaire du monomère (ou du monomère le plus lourd s'il y a plusieurs monomères) desdits monomères isocyanates ;
(c) au moins 15% et au plus 60% en masse par rapport à la masse totale des composants a), b) et c), d'un mélange de composés polyisocyanates issus desdits ou dudit monomère(s) (c'est-à-dire porteur d'au moins de 2 fonctions isocyanates) distincts de a) et de b) ;
(d) au moins 1%, avantageusement 3% en masse par rapport à la masse des composants a), b), c), d), de composés porteurs d'au moins une fonction isocyanate et porteur d'au moins un motif choisi parmi les 6-amino-5-aza-uracile et 2-imino-4-oxo-1,3-diazétidine ;
(e) de 0 à 10% en masse par rapport à la masse des composants a), b), c), d) et e) d'impuretés y compris les monomères de départ ;
et
(f) de 0 à 100%, avantageusement de 0 à 70% en masse par rapport à la masse des composants a), b), c), d) et e) d'un solvant (c'est-à-dire inerte vis à vis des isocyanate) dont le point d'ébullition sous pression atmosphérique est avantageusement au plus égal à 200°C, de préférence à 150°C.

Dans d) les composés à motif 6-amino-5-aza-uracile ne dépassent avantageusement pas 20 %, de préférence pas 10% de a), b), c) et d). En revanche, les composés à motifs 2-imino-4-oxo-1,3-diazétidine peuvent atteindre 90%, voire 95% de a), b), c) et d). Toutefois, lorsque les composés à motifs 2-imino-4-oxo-1,3-diazétidine dépassent 40%, le rapport massique entre composés présentant plus d'un motif 2-imino-4-oxo-1,3-diazétidine de composés et ceux en comportant un seul est alors avantageusement au moins égal à 1/10.

Le plus souvent a) représente au moins 1% en masse par rapport à la masse totale des composants a), b) et c), et selon un mode avantageux de la présent invention au moins 5% et même au moins 10%.

Les composants a) + b) représentent avantageusement au moins 10%, de préférence au moins 20% de composants a), b) et c).

Les composants b) + d) représentent avantageusement au moins 30%, de préférence 40%, plus préférentiellement 50% en masse de a), b), c) et d).

Il est également intéressant que les composés à motifs 2-imino-4-oxo-1,3-diazétidine représentent au moins 20% avantageusement 30% de préférence au moins 40% de a), b), c) et d).

Ces compositions sont avantageusement à base de monomère choisi parmi le HDI, le NBDI, l'IPDI et leurs mélanges.

Le composant (f) est avantageusement au plus égal à 5%, de préférence à 3%.

Les monomères de départ représentent avantageusement au plus 10%, de préférence au plus 2% des composants a), b), c), d) et e).

La présente invention vise également les compositions comportant par addition successive ou simultanée :

- une composition isocyanate présentant des fonctions isocyanates, libres ou masquées, avantageusement aliphatiques ;
- un composé salin selon l'invention ou un composé issu de l'addition d'un composé salin selon l'invention avec une fonction isocyanate.

Ladite composition isocyanate comporte avantageusement un monomère ou un mélange de monomères, telle que décrite ultérieurement.

Le rapport molaire entre quantité catalyseur (exprimée en mole d'anion de formule (I)) / quantité fonctions NCO (exprimée en équivalent) est avantageusement d'au moins $10^{-5}$, et d'au plus $5.10^{-2}$, de préférence dans l'intervalle fermé, c'est-à-dire comprenant les bornes allant de $10^{-4}$ à $10^{-3}$. Il convient de choisir dans la partie basse de la fourchette les alcalins, alcalino-terreux et les oniums et les iniums, c'est-à-dire de $10^{-5}$ à $5.10^{-3}$, avantageusement de $5.10^{-5}$ à.$10^{-3}$

et pour les autres cations dans la partie haute de la fourchette, c'est-à-dire de $10^{-4}$ à $5.10^{-2}$, avantageusement de $5.10^{-4}$ à $5.10^{-3}$.

Lorsque la fonction isocyanate à (cyclo)condenser est une fonction portée par un radical primaire aliphatique non néopentylique, les valeurs maximales ci dessus peuvent être divisées par un facteur cinq, voire dix.

L'invention a également pour objet un procédé de (cyclo)condensation catalytique d'isocyanates monomères qui comprend :

a) la réaction des isocyanates monomères de départ avec un catalyseur comprenant un composé salin minéral ou organique d'un composé de formule (I) et notamment de formules (1) et (2) :

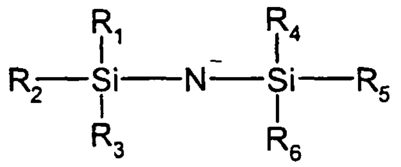

(1)

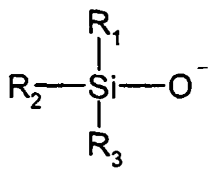

(2)

dans lesquelles les symboles $R_1$, $R_2$, $R_3$, $R_4$, $R_5$ et $R_6$, identiques ou différents, représentent un groupe monovalent de nature hydrocarbonée ayant de 1 à 30 atomes de carbone, aliphatique, cycloaliphatique saturé ou insaturé, aryle, aralcoyle ou alcoylaryle, éventuellement substitué par un ou plusieurs atomes d'halogène, groupements CN, ou ester, et/ou éventuellement interrompu par un ou plusieurs atomes choisis parmi O, S, N et Si.

Dans la formule (1) l'un de $R_1$, $R_2$, $R_3$, $R_4$, $R_5$ et $R_6$ représente un motif de formule :

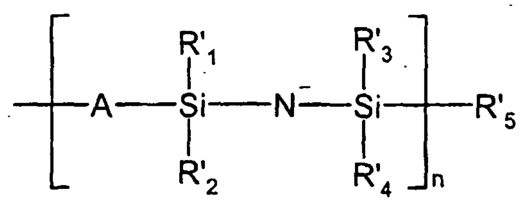

- A étant une chaîne alcoylène ayant de 1 à 30 atomes, avantageusement de 2 à 20 atomes de carbone éventuellement substituée par un ou plusieurs atomes d'halogène, groupements CN, ou ester, et/ou éventuellement interrompue par un ou plusieurs atomes choisis parmi O, S, N et Si, de préférence $(CH_2)_{n'}$ avec n' compris entre 1 et 6, avantageusement de 2 à 6,
- et $R'_1$ à $R'_5$, identiques ou différents, représentant un groupe monovalent de nature hydrocarbonée, aliphatique, cycloaliphatique saturé

ou insaturé, aryle, aralcoyle ou alcoylaryle, éventuellement substitué par un ou plusieurs atomes d'halogène, groupements CN, ou ester, et/ou éventuellement interrompu par un ou plusieurs atomes choisis parmi O, S, N et Si, et

- n est un nombre entier entre 1 et 50,
- ou deux parmi $R_1$, $R_2$ et $R_3$ d'une part et/ou $R_4$, $R_5$ et $R_6$ d'autre part, constituent ensemble un groupe hydrocarboné drivalent, éventuellement substitué par un ou plusieurs atomes d'halogène, groupements CN, ou ester, et/ou éventuellement interrompu par un ou plusieurs atomes choisis parmi O, S, N et Si, et/ou
- au moins un groupe choisi parmi $R_1$, $R_2$ et $R_3$ forme avec au moins un groupe parmi $R_4$, $R_5$ et $R_6$ un groupe hydrocarboné divalent, éventuellement substitué par un ou plusieurs atomes d'halogène, groupements CN, ou ester, et/ou éventuellement interrompu par un ou plusieurs atomes choisis parmi O, S, N et Si, ou un précurseur dudit composé,

b) à une température d'au moins 20°C et avantageusement d'au moins 40°C et d'au plus 200°C, avantageusement d'au plus 150°C,

c) l'arrêt de la réaction de (cyclo)condensation au taux de transformation souhaité compris avantageusement entre 5 et 95%, de préférence entre 10 et 50% ; et

d) l'élimination éventuelle des monomères n'ayant pas réagi ou la réaction avec d'autres composés réactifs avec la fonction isocyanate.

Avantageusement $R_1$ à $R_6$ et $R'_1$ à $R'_5$ identiques ou différents représentent :

- un groupe alcoyle, alcényle, halogénoalcoyle ou halogénoalcényle ayant de 1 à 20, de préférence de 1 à 6 atomes de carbone, éventuellement substitué par un ou plusieurs atomes de chlore et/ou de fluor, et/ou éventuellement interrompu par un ou plusieurs atomes d'azote et/ou de silicium,
- un groupe cycloalcoyle, cycloalcényle, halogénocycloalcoyle

ou halogénocycloalcényle ayant de 3 à 30, de préférence 3 à 10 atomes de carbone et contenant des atomes de chlore et/ou de fluor, et/ou éventuellement interrompu par un ou plusieurs atomes d'azote et/ou de silicium,

- un groupe aryle, alcoylaryle ou halogénoaryle ayant de 6 à 30, de préférence 6 à 10 atomes de carbone et contenant des atomes de chlore et/ou de fluor,
- un groupe cyanoalcoyle ayant de 1 à 6 atomes de carbone,
- ou deux groupes parmi $R_1$, $R_2$ et $R_3$ ou $R_3$, $R_4$ et $R_5$ forment ensemble un radical divalent comprenant de 2 à 5 atomes de carbone, et/ou éventuellement interrompu par un ou plusieurs atomes d'azote et/ou de silicium,
- ou deux parmi $R_1$, $R_2$ et $R_3$ d'une part et/ou $R_4$, $R_5$ et $R_6$ d'autre part, constituent ensemble un groupe hydrocarboné divalent et/ou éventuellement interrompu par un ou plusieurs atomes d'azote et/ou de silicium, ou
- au moins un groupe choisi parmi $R_1$, $R_2$ et $R_3$ forme avec au moins un groupe parmi $R_4$, $R_5$ et $R_6$, un groupe hydrocarboné divalent, comprenant de 2 à 5 atomes de carbone, et/ou éventuellement interrompu par un ou plusieurs atomes d'azote et/ou de silicium.

De préférence, $R_1$ à $R_6$ identiques ou différents sont choisis parmi les groupes méthyle, éthyle, propyle, isopropyle, butyle, isobutyle, $\alpha$-pentyle, t-butyle, chlorométhyle, dichlorométhyle, $\alpha$-chloro-éthyle, $\alpha,\beta$-dichloro-éthyle, fluorométhyle, difluorométhyle, $\alpha,\beta$-difluoro-éthyle, trifluoro-3,3,3-propyle, trifluorocyclopropyle, trifluoro-4,4,4-butyle, heptafluoro-3,3,3,4,4,5,5-pentyle, $\beta$-cyano-éthyle, $\gamma$-cyanopropyle, phényle, p-chlorophényle, m-chlorophényle, dichloro-3,5-phényle, trichlorophényle, tétrachlorophényle, o-, p-, ou m-tolyle, $\alpha,\alpha,\alpha$-trifluorotolyle ; et xylyles (diméthyl-2,3-phényle ; diméthyl-3,4-phényle) ;
ou deux groupes parmi $R_1$, $R_2$ et $R_3$, d'une part et/ou $R_4$, $R_5$ et $R_6$, d'autre part pris ensemble représentent un groupe éthylène, propylène, butylène, pentylène, de préférence leurs isomères linéaires, triméthylène, tétraméthylène ou pentaméthylène, ou un groupe $-N^{-}-Si(CH_3)_2-N^{-}-$ ,
et/ou $R_1$ et $R_4$, pris ensemble, représentent un groupe choisi parmi. éthylène, propylène, butylène, pentylène, de préférence les isomères non ramifiés, diméthylène et triméthylène.

Des groupes $R_1$ à $R_6$ et $R'_1$ à $R'_5$ particulièrement préférés sont choisis parmi méthyle, éthyle, propyle, linéaires ou ramifiés le cas échéant, vinyle et phényle, pouvant éventuellement être chloré et/ou fluoré. Le cas du vinyle est intéressant dans la mesure où ce type de composé peut permettre la polymérisation ou la copolymérisation avec d'autre monomère éthylénique et donc permet l'insertion du motif catalyseur (en particulier les motifs des formules (1), (2), (Va) et (Vb) où l'un au moins des groupes $R_1$ à $R_6$ assure la liaison avec le polymère) dans un réseau polymérique.

Lorsque les groupes $R_1$ à $R_6$ et $R'_1$ à $R'_5$ sont chlorés et/ou fluorés, le nombre d'atomes d'halogène varie de 1 à la totalité des valences disponibles.

On peut tout particulièrement citer les sels des composés silazanes suivants :

hexaméthyldisilazane,
diéthyl 1-3, tétraméthyl-1,1,3,3 disilazane,
divinyl-1,3 tétraméthyl-1,1,3,3 disilazane,
hexaéthyldisilazane,
diphényl-1,3 tétraméthyl-1,1,3,3 disilazane,
hexaméthylcyclotrisilazane, et
éthylène-2,5-tétraméthyl 2,2,5,5-cyclodisilazane.

On peut également citer les silanolates suivants :

triméthylsilanolate,
triéthylsilanolate,
éthyldiméthylsilanolate,
vinyldiméthylsilanolate.

Le sel du composé de formules (I), notamment (1), (2), (Va) ou (Vb), peut être un sel minéral, monovalent ou multivalent, ou un mélange de ces sels.

Les sels minéraux, avantageusement bien dissociés, préférés sont les alcalins et alcalinoterreux, notamment ceux de K, Li, Na et Mg.

Le sel du composé de formule (I), notamment (1), (2), (Va) ou (Vb), peut également être un sel organique, monovalent ou multivalent ou un mélange de ces sels. Les sels organiques préférés sont les "oniums" ou "iniums" stables.

Selon une alternative avantageuse de l'invention, le sel du composé de formule (I) est un sel d'erbium du composé de formule (1), et on préfère dans ce cas que tous les ligands soient des sels d'erbium du composé de formule (1). Ceux-ci répondent à la formule générale ($1^E$) suivante, lorsque n' représente zéro :

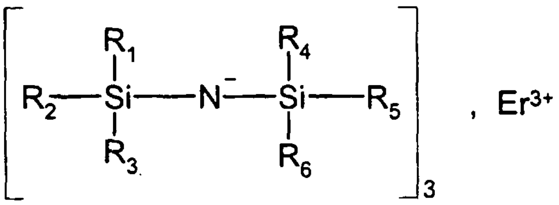

($1^E$)

On peut tout particulièrement citer les sels d'erbium des composés suivants :

hexaméthyldisilazane,
diéthyl-1,3-tétraméthyl-1,1,3,3-disilazane,
divinyl-1,3-tétraméthyl-1,1,3,3-disilazane,
hexaéthyldisilazane, et
diphényl-1,3-tétraméthyl-1,1,3,3-disilazane.

Selon une autre alternative avantageuse de l'invention, le sel du composé de formule (I) est un sel d'étain (II) du composé de formule (1), et on préfère dans ce cas que tous les ligands soient des sels d'étain du composé de formule (1). Ceux-ci répondent à la formule générale ($1^S$) suivante, lorsque n' représente zéro :

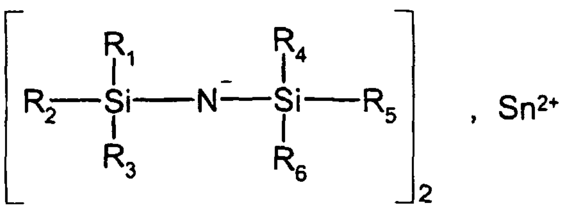

($1^S$)

On peut tout particulièrement citer les sels d'étain (II) des composés suivants :

hexaméthyldisilazane,
diéthyl-1,3-tétraméthyl-1,1,3,3-disilazane,
divinyl-1,3-tétraméthyl-1,1,3,3-disilazane,
hexaéthyldisilazane,
diphényl-1,3-tétraméthyl-1,1,3,3-disilazane,
hexaméthylcyclotrisilazane, et
éthylène-2,5-tétraméthyl 2,2,5,5-cyclodisilazane.

Dans le cas de cations multivalents, le sel selon l'invention peut comprendre au moins un anion, souvent jouant

le rôle de coordinant (ou ligand), de formule (I), notamment (1), (2), (Va) ou (Vb), ci-dessus et éventuellement un ou plusieurs anions, souvent jouant le rôle de coordinants (ou ligands), différents.

Généralement, on préfère que tous les anions, souvent jouant le rôle de coordinants (ou ligands), soient des composés de formule (1) ou (2).

Le nombre des anions, souvent jouant le rôle de coordinants (ou ligands), est fonction de la valence du cation minéral ou organique, ainsi que du nombre d'atomes d'azote dans le composé de formule (1).

Les oniums sont choisis dans le groupe des cations formés par les éléments des colonnes VB et VIB (tels que définis dans le tableau de la classification périodique des éléments, publié au supplément du Bulletin de la Société Chimique de France en janvier 1966) avec 4 (cas de la colonne VB) ou 3 (cas de la colonne VIB) chaînes hydrocarbonées.

Avantageusement, le sel organique de l'invention est dans ce cas un phosphonium, sulfonium ou ammonium.

Les iniums, ensemble auquel appartiennent les pyridiniums, dérivent des oniums par le remplacement de deux substituants par un substituant doublement lié.

On peut également avoir un mélange de sels minéraux et organiques.

Les sels de silazane de l'invention sont des composés connus disponibles dans le commerce ou peuvent être aisément préparés à partir des silazanes correspondants, par exemple par réaction avec un amidure ou hydrure métallique tels que LiH, NaH, $NaNH_2$, KH, ou un alcoyl-métal, par exemple le butyllithium en milieu éther à température ambiante.

Les sels de l'invention peuvent également être obtenus par échange du cation lithium d'un sel de lithium avec un cation de métal lourd, par exemple l'erbium ou l'étain, au moyen du chlorure correspondant, par exemple le chlorure d'erbium ou le chlorure d'étain

Les silazanes de départ sont des composés connus disponibles dans le commerce ou peuvent être obtenus à partir des chlorosilazanes correspondants, au moyen de réactions simples usuelles dans le domaine en question.

Pour la préparation des silazanes cycliques, on pourra se reporter à Houben-Weyl, Methoden der Organischen Chemie : "Organo-silicum Verbindungen", Georg Thieme Verlag, Stuttgart, (1980).

Pour plus de précisions concernant la préparation des sels de silazane, on pourra se reporter au même ouvrage ou se référer aux méthodes décrites dans Organomet. Chem., (1983), 461(1-2), 75-80 ; Chem. Prum., (1977), 27(11), 566-8 ; Zh. Obstich. Khim., (1988), 58(8), 934-5 ; Inorg. Synth., (1966), 8, 19-22 ou encore Inorg. Chem., (1966), 8, 15-19.

Les silanolates de l'invention sont des composés connus disponibles dans le commerce ou peuvent être obtenus à partir des chlorosilanes correspondants, en milieu basique ou par échange du cation $Li^+$ avec un sel d'un métal lourd, tel que l'erbium ou l'étain, par exemple.

Lorsque le composé de formule (I), notamment (1), (2), (Va) ou (Vb), est formé *in situ,* on part d'un précurseur dudit composé qui est libéré par un moyen connu en soi, pour former le composé actif correspondant.

Dans l'étape c), les autres composés réactifs avec la fonction isocyanate peuvent être des isocyanates ou des composés porteurs d'un atome d'hydrogène mobile, notamment les amines, alcools, imidazoles, pyrazoles, malonates, etc., substitués ou non substitués.

Ainsi, les composés 2-imino-4-oxo-1,3-diazétines (IUT), obtenus lors de l'utilisation des sels d'étain (II) dans le procédé selon l'invention, conduisent en présence d'acide chlorhydrique, par réaction avec un composé isocyanate, notamment dans lequel la fonction isocyanate est portée par un atome de carbone appartenant à un cycle aromatique, à des dérivés asymétriques à cycle 4,6-dioxo-2-imino-hexahydro-1,3,5-triazine de formule :

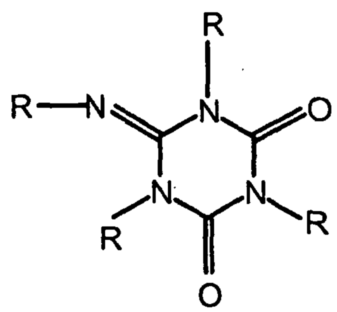

dans lequel R est tel que défini pour les composés à structure IUT ci-dessus.

Les catalyseurs de l'invention sont appropriés à la l'oligo- et à la polycondensation, notamment l'oligomérisation et en particulier la (cyclo)trimérisation des isocyanates aliphatiques linéaires, ramifiés, notamment en $\alpha$ ou $\beta$, cycloaliphatiques ou aromatiques.

La présente invention vise l'oligomérisation, notamment la trimérisation de composés isocyanates en général et, de préférence, porteurs de deux fonctions isocyanates, désignés dans la présente description par isocyanates monomères.

Il peut s'agir de monomères isocyanates à squelette hydrocarboné exclusivement de nature aliphatique, linéaire, ramifiés ou cycliques ou d'isocyanates aromatiques.

On peut citer en particulier comme monomère aliphatique (c'est-à-dire dont le carbone porteur de l'azote de la fonction isocyanate est d'hybridation $sp^3$) les primaires non néopentyliques et plus spécifiquement les linéaires vrais tel que l'hexaméthylènediisocyanate (HDI) et le tetraméthylènediisocyanate.

On peut citer également les monomères aliphatiques dont le squelette hydrocarboné est ramifié mais dont les fonctions isocyanates sont portées par des atomes de carbone primaire, par exemple le 2-méthylpentanediisocyanate.

On peut encore citer les monomères dont au moins une fonction isocyanate est en position secondaire, notamment cycloaliphatique, tertiaire ou néopentylique. Toutefois ces fonctions isocyanates sont, en règle générale, moins réactives que les primaires et demandent, lorsque les co-cations sont des cations ne facilitant pas la dissociation et notamment lorsqu'ils sont di- ou polyvalents, des conditions plus dures (teneur en catalyseur plus importante et température plus élevée d'au moins 20°C, et même de 30°C, voire 40°C).

Ainsi, parmi ces produits, ceux qui donnent d'excellents résultats avec les catalyseurs de l'invention, à la différence des autres catalyseurs avec lesquels ils n'ont qu'un taux de transformation médiocre, sont les monomères dans lesquels la fonction isocyanate est portée par un atome de carbone cycloaliphatique, secondaire, tertiaire ou néopentylique, en particulier les isocyanates cycloaliphatiques. Ces monomères sont tels qu'au moins l'une avantageusement des deux fonctions isocyanates est distante du cycle le plus proche d'au plus un carbone et, de préférence, est reliée directement à lui. En outre, ces monomères cycloaliphatiques présentent avantageusement au moins une, de préférence deux fonctions isocyanates choisies parmi les fonctions isocyanates secondaire, tertiaire ou néopentylique.

De manière surprenante, on obtient de bons résultats pour des isocyanates dans lesquels la liberté conformationnelle du carbone portant la fonction NCO est faible. On peut citer à titre d'exemple les monomères suivants :

- les composés correspondant à l'hydrogénation du ou des noyaux aromatiques porteurs des fonctions isocyanates de monomères d'isocyanates aromatiques et notamment du TDI (toluènediisocyanate) et des diisocyanato-biphényles, le composé connu sous le sigle $H_{12}$MDI (4,4'-bis(isocyanatocyclohexyl)méthane), les divers BIC [bis(isocyanatométhylcyclohexane)] et les cyclohexyldi-isocyanates éventuellement substitués ;
  et surtout
- le norbornanediisocyanate, souvent désigné par son sigle NBDI ;
- l'isophoronediisocyanate, ou IPDI, ou plus précisément 3-isocyanatométhyl-3,5,5-triméthylcyclo-hexylisocyanate.

Comme monomères aromatiques, on peut citer les :

- 2,4- ou 2,6- toluène diisocyanate (TDI) ;
- 2,6-4,4'-diphénylméthane diisocyanate (MDI) ;
- 1,5-naphtalène diisocyanate (NDI) ;
- tolidine diisocyanate (TODI) ;
- p-phénylène diisocyanate (PPDI).

Les monomères de départ peuvent également être des produits d'oligomérisation d'isocyanates de plus faible masse moléculaire, ces produits d'oligomérisation étant porteurs de fonctions isocyanates. Dans ce cas, il n'est pas nécessaire de séparer l'oligomère non transformé du produit réactionnel formé à l'issue de la réaction de trimérisation.

Le catalyseur est de préférence utilisé tel quel ou dissous dans un solvant inerte avant introduction dans le milieu réactionnel. Le solvant de formulation du catalyseur est de préférence inerte vis-à-vis des isocyanates du milieu réactionnel. Dans le cas où le solvant est réactif vis-à-vis des isocyanates, on ajuste la concentration du catalyseur de telle manière que la quantité de solvant ne soit pas préjudiciable à l'obtention du composé de l'application visée.

Le solvant est de préférence choisi de façon à ce que son point d'ébullition soit supérieur à la température de réaction. On peut citer les hydrocarbures aliphatiques tels que l'hexane ou aromatiques tels que le toluène.

On peut toutefois travailler sous pression avec des solvants ou des gaz à l'état supercritique.

La quantité de catalyseur varie en fonction de la nature du cation métallique du sel de silazane, et de l'isocyanate de départ.

Ainsi, la réactivité des sels de silazane suit l'ordre suivant :

$$K+ \geq Na+ > Li+.$$

Le ratio molaire quantité catalyseur / quantité fonctions NCO est avantageusement d'au moins $5.10^{-5}$, et d'au

plus $5.10^{-3}$, de préférence entre $10^{-4}$ et $10^{-3}$.

La quantité de catalyseur est avantageusement d'environ 0,01 à 0,07% rapportée au poids du cation.

Lorsque les gaz dissous dans les isocyanates monomères de départ sont éliminés, la quantité de catalyseur peut être réduite.

En outre, lorsque l'on élimine les gaz dissous dans les isocyanates monomères de départ, la réactivité des catalyseurs est améliorée.

Les gaz dissous ($CO_2$, gaz halogénés, $O_2$, ...) peuvent être éliminés par tout moyen connu, notamment par barbotage d'un gaz inerte tel que l'azote ou l'argon, ou par mise sous vide du milieu réactionnel.

On choisira donc la quantité de catalyseur en fonction de la cinétique de réaction et des propriétés physico-chimiques (viscosité, etc.) attendues, des quantités trop faibles ne permettant pas l'obtention de trimères en quantité notable mais donnant des produits de faible viscosité avec un taux de trimères vrais (un seul motif isocyanurate) élevé. À l'inverse, des quantités trop élevées risquent de créer un emballement de la réaction.

La température de réaction est d'au moins 20°C, avantageusement d'au moins 30°C.

Dès que la réaction démarre, elle est exothermique. Il est donc généralement préférable de contrôler la température du milieu réactionnel pour éviter un emballement de la réaction susceptible de conduire à une polymérisation non contrôlée de l'isocyanate.

Il est généralement préférable d'introduire le catalyseur dilué progressivement par exemple par coulée lente.

Dans certains cas, lorsque l'on cherche à obtenir des composés notamment des (cyclo)trimères à fonction urée ou biuret, on peut être amené à ajouter de l'eau au milieu réactionnel.

Cependant, on a observé que l'eau ajoutée conduisait à un ralentissement de la cinétique de (cyclo)condensation, notamment de (cyclo)trimérisation.

La réaction de polycondensation est stoppée au taux de transformation voulu, généralement entre 5% et 95%, avantageusement entre 8% et 60% des fonctions NCO, par addition d'un désactivateur du catalyseur ou par élimination de celui-ci par tout moyen connu de l'homme du métier, tel qu'absorption sur colonne, etc.

On peut, notamment, stopper la réaction par addition d'un composé acide (par exemple, un sel d'amine) ou un composé ou un mélange de composés pouvant générer dans le milieu réactionnel un composé acide susceptible de neutraliser l'activité du catalyseur, notamment un sel d'acide.

Au cours de l'étude qui a mené à la présente invention, il a été montré qu'il était préférable d'ajouter un acide de force élevée, généralement ayant un $pKa \leq 3$, tel que les acides sulfuriques ou sulfoniques, par exemple les acides mésylique, tosylique, ou halogénosulfoniques, ou un acide phosphorique, ou phosphoniques de pKa plus élevée, de préférence un ester d'acide phosphoré.

Comme esters d'acides phosphorés, on peut citer les esters de phosphate, notamment les mono-esters, diesters ou leurs mélanges.

On peut citer les phosphonates, de préférence monoestérifiés, et les phosphinates.

On peut également citer les acides minéraux, de préférence halogénés, par exemple HF, HCl, HBr.

L'empoisonnement du catalyseur est toutefois de préférence réalisé au moyen d'esters acides de l'acide phosphorique, et notamment des esters phosphoriques de dialcoyle, en particulier le phosphate de dibutyle et le phosphate de di-2-éthylhexyle.

On peut aussi utiliser des composés précurseurs d'un composé acide tels que des acides de Lewis ($AlCl_3$, $ZnCl_2$) ou des triflates métalliques.

Le produit réactionnel contient des produits de polycondensation, notamment de (cyclo)condensation des isocyanates de départ.

Il s'agit principalement, voire exclusivement, de composés à cycle isocyanurate, notamment de trimères "vrais" possédant un seul cycle isocyanurate, en particulier lorsque le cation est un cation organique du type décrit précédemment ou un cation minéral tel que $K^+$, $Na^+$, $Li^+$.

Il peut s'agir également de composés trimères ayant un cycle différent de l'isocyanurate. Par exemple, lorsque le cation du composé de formule (I) employé est l'erbium, les trimères obtenus dans la réaction de (cyclo)condensation peuvent contenir un cycle 6-amino-5-azauracile de formule:

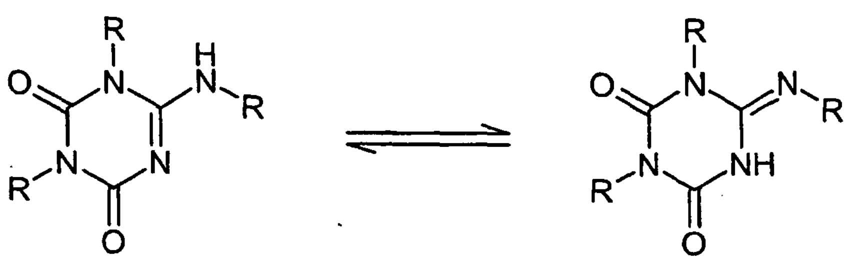

dans laquelle R représente le reste d'un composé à fonction isocyanate, après enlèvement de la dite fonction isocyanate.

À l'issue du procédé tel que défini ci-dessus et lorsque des sels d'étain, notamment d'étain (II), sont utilisés comme cations des composés de formule (I), on peut obtenir une composition comprenant principalement, outre les isocyanates monomères de départ n'ayant pas réagi, des composés dimères, notamment cyclodimères. Les cyclodimères obtenus possèdent un seul cycle de structure 2-imino-4-oxo-1,3-diazétidine (IUT) de formule :

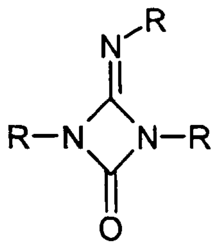

dans laquelle R est le reste d'un composé monomère isocyanate de formule R-N-CO, dans laquelle R est notamment un reste hydrocarboné aliphatique, linéaire ou ramifié, cycloaliphatique ou aromatique ayant de 4 à 30 atomes de carbone comportant éventuellement un ou plusieurs substituants choisis parmi un groupe NCO, un groupe carbamate, allophanate, ester ,éther, etc.

Il peut s'agir également de composés oligomères ayant deux voire plus de deux cycles 2-imino-4-oxo-1,3-diazétidine (IUT).

De tels composés comportant un cycle 2-imino-4-oxo-1,3-diazétidine sont déjà connus, notamment pour avoir été préparés par cycloaddition (2 + 2) d'isocyanates sur des carbodiimides (Houben Weyl, Methoden der Organischen Chemie, 4. Auflage, Georg Thieme Verlag, (1983), 1111-1113).

La présence d'une teneur substantielle en composés de structure IUT dans une composition polyisocyanate est particulièrement intéressante dans la mesure où ces composés ont une fonctionnalité NCO égale à 3 et une masse plus faible en comparaison des monoisocyanurates correspondants de même fonctionnalité.

De manière générale, lorsque est mis en oeuvre le procédé selon l'invention et selon le taux de transformation, le mélange final peut comprendre en outre des composés lourds de plus haut poids moléculaire.

L'invention a également pour objet une composition isocyanate susceptible d'être obtenue par le procédé tel que défini ci-dessus.

Cette composition est notamment caractérisée en ce qu'elle comprend moins de 1% en poids d'isocyanates monomères par rapport au poids total des composés isocyanates présents dans la composition. Elle peut avantageusement être caractérisée par le fait que la concentration en fonction isocyanate libre est comprise entre 0,5 et 7 équivalents par litre.

De plus, la composition isocyanate selon l'invention comporte en masse au moins 1%, avantageusement au moins 3%, de préférence au moins 5% de composé présentant au moins un motif :

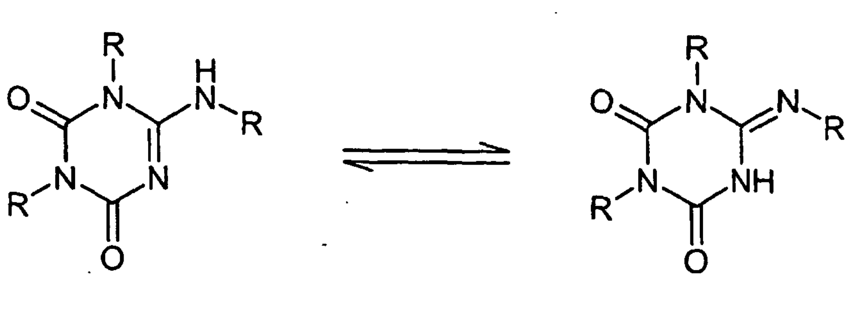

où les R, identiques ou différents, représentent les restes du, ou des monomères, après ignorance d'une des fonctions isocyanates, les autres pouvant être engagées dans une condensation distincte.

Selon un autre aspect, la composition isocyanate selon l'invention comporte en masse au moins 1%, avantageusement au moins 3%, de préférence au moins 5% de composé présentant au moins un motif :

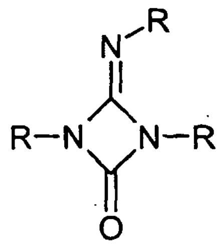

où les R, identiques ou différents, représentent les restes du, ou des monomères, après ignorance d'une des fonctions isocyanates, les autres pouvant être engagées dans une condensation distincte.

En particulier, lorsque le procédé a été mis en oeuvre avec des sels d'erbium, la composition est caractérisée en ce qu'elle comprend avantageusement :

- de 50 à 80% en poids de composés trimères consistant en mono-isocyanate et 6-amino-5-aza-uraciles,
- de 0,5 à 5%, avantageusement de 0,5 à 3% de composés mono-urétidiones,
- moins de 1 % en poids d'isocyanates monomères,

par rapport au poids total des composés isocyanates présents dans la composition.

Parallèlement, lorsque le procédé est mis en oeuvre avec des sels d'étain, la composition est caractérisée en ce qu'elle comprend avantageusement :

- de 50 à 75 %, avantageusement de 55 à 70 % en poids de composés mono-2-imino-4-oxo-1,3-diazétidine ;
- de 10 à 25 % en poids, avantageusement de 12 à 20 % en poids de composés bis-(2-imino-4-oxo-1,3-diazétidine) ;
- de 10 à 25 %, avantageusement de 14 à 20 % en poids d'isocyanates-urées ; et
- moins de 1 % en poids d'isocyanates monomères,

par rapport au poids total des composés isocyanates présents dans la composition.

L'invention a également pour objet une composition polyisocyanate masquée, caractérisée en ce qu'elle comprend moins de 1% en poids d'isocyanates monomères par rapport au poids total des composés isocyanates présents dans la composition, ladite composition comprenant de 0,5 à 100 %, avantageusement de 10 à 100 %, de préférence de 20 à 100 % de fonctions isocyanates masquées à l'aide d'un agent masquant de la fonction isocyanate.

En particulier, lorsque le procédé a été mis en oeuvre avec des sels d'erbium, la composition polyisocyanate masquée est caractérisée en ce qu'elle comprend avantageusement :

- de 50 à 80% en poids de composés trimères consistant en mono-isocyanates et 6-amino-5-aza-uraciles,
- de 0,5 à 5%, avantageusement de 0,5 à 3% de composés mono-urétidiones,
- moins de 1 % en poids d'isocyanates monomères,

par rapport au poids total des composés isocyanates présents dans la composition, ladite composition comprenant de 0,5 à 100%, avantageusement de 10 à 100%, de préférence de 20 à 100% de fonctions isocyanates masquées à l'aide d'un agent masquant de la fonction isocyanate.

Parallèlement, lorsque le procédé est mis en oeuvre avec des sels d'étain, la composition polyisocyanate masquée est caractérisée en ce qu'elle comprend avantageusement :

- de 50 à 75 %, avantageusement de 55 à 70 % en poids de composés mono-2-imino-4-oxo-1,3-diazétidine ;
- de 10 à 25 % en poids, avantageusement de 12 à 20 % en poids de composés bis-(2-imino-4-oxo-1,3-diazétidine) ;
- de 10 à 25 %, avantageusement de 14 à 20 % en poids d'isocyanates-urées ; et
- moins de 1 % en poids d'isocyanates monomères,
  par rapport au poids total des composés isocyanates présents dans la composition, ladite composition comprenant de 0,5 à 100%, avantageusement de 10 à 100%, de préférence de 20 à 100 % de fonctions isocyanates masquées à l'aide d'un agent masquant de la fonction isocyanate.

L'agent masquant, qui peut être un mélange d'agents masquants, présente le plus souvent au moins un hydrogène mobile de manière que la réaction de masquage puisse s'écrire :

$$Is\text{-}N{=}C{=}O + AM\text{-}H \rightarrow Is\text{-}NH\text{-}CO(AM)$$

ou AM-H représente l'agent masquant, AM- représente le groupe masquant, Is est le reste porteur de la fonction isocyanate considérée, notamment d'une composition telle que définie ci-dessus.

Ledit agent masquant présente au moins une fonction portant un hydrogène mobile, ou plus exactement réactif, fonction pour laquelle on peut définir un pKa lequel correspond, soit à l'ionisation d'un acide, y compris l'hydrogène des fonctions, phénols et alcools, soit à l'acide associé d'une base, en général azotée. Le pKa de la fonction présentant des hydrogènes est au moins égal à 4, avantageusement à 5, de préférence à 6 et est au plus égal à 14, avantageusement à 13, de préférence à 12, et de manière plus préférée à 10, une exception devant être faite pour les lactames dont le pKa est supérieur à ces valeurs et qui constituent des agents masquants néanmoins acceptables bien que non préférés pour l'invention. Avantageusement, l'agent masquant ne comporte qu'un seul hydrogène mobile.

A titre d'exemples non limitatifs, des agents de masquage selon l'invention, on peut citer les dérivés de l'hydroxylamine tels que l'hydroxysuccinimide et les oximes telles tel que la méthyléthylcétoxime, les dérivés des phénols ou assimilés, les dérivés des amides tels les imides et les lactames, ainsi que les malonates ou céto-esters et les hydroxamates.

On peut également citer les groupes hétérocycliques azotés comprenant 2 à 9 atomes de carbone et, outre l'atome d'azote, de 1 à 3 autres hétéroatomes choisis parmi l'azote, l'oxygène et le soufre. On préfère en particulier les hétérocycles comportant de 2 à 4 atomes de carbone et de 1 à 3 atomes d'azote, tels les groupes pyrrolyle, 2H-pyrrolyle, imidazolyle, pyrimidinyle, pyridazinyle, pyrazinyle, pyrimidinyle, pyridazinyle; indolizinyle, iso-indolyle, indolyle, indolyle, indazolyle, purinyle, quinolizinyle, isoquinolyle, pyrazolidinyle, imidazolidinyle et triazolyle, ces groupes étant éventuellement substitués par un à trois substituant choisis parmi $NH_2$,NH(alkyle en $C_1$-$C_6$), N-(dialcoyle en $C_1$-$C_6$), OH, SH, $CF_3$, alcoyle en $C_1$-$C_6$, cycloalcoyle en $C_3$-$C_6$, aryle en $C_5$-$C_{12}$, notamment phényle, aralcoyle en $C_6$-$C_{18}$ ayant de 5 à 12 atomes de carbone dans le groupe aryle, notamment benzyle ou alcoylaryle, en $C_6$-$C_{18}$ ayant de 5 à 12 atomes de carbone dans le groupe aryle.

Pour la détermination des pKa, on pourra se reporter à "The determination of ionization constants, a laboratory manual", A. Albert, E. P. Serjeant, Chapman and Hall Ltd, London. Pour la liste des agents bloquants, on pourra se reporter à Z. Wicks (Prog. Org. Chem., (1975), 3, 73, et Prog. Org. Chem., (1989), 9,7) et Petersen (Justus Liebigs, Annalen der Chemie, (1949), 562, 205).

Les compositions polyisocyanates masquées selon l'invention sont obtenues par réaction du produit de l'étape c) ou d) avec un agent masquant tel que défini ci-dessus dans les conditions habituelles.

Lorsque l'agent masquant est mis à réagir avec le produit de l'étape c), il est opportun de réaliser une étape d) d'élimination des isocyanates monomères masqués, le cas échéant à l'issue de l'étape de masquage proprement dite.

Les compositions selon l'invention présentent l'avantage de comprendre des oligomères (notamment dimères et trimères) d'isocyanates, dont l'effet est d'abaisser substantiellement la viscosité de la composition finale.

Les compositions ainsi obtenues présentent en outre l'avantage d'un séchage plus rapide lors de leur utilisation pour la réalisation de revêtements, notamment de peintures ou vernis.

Les compositions selon l'invention sont avantageusement utilisées comme durcisseur de compositions pour revêtements, notamment de peintures ou de vernis, par réaction des fonctions isocyanates libres ou libérées par départ de l'agent masquant avec un composé réactif avec la liaison isocyanate multifonctionnelle, notamment un polyol.

Les compositions selon l'invention peuvent également être utilisées dans des adhésifs, ou encore pour la micro-encapsulation de matières actives encapsulables, notamment des encres, biocides, détergents, etc.

Les exemples suivants sont destinés à illustrer l'invention. Sauf indications contraires, les pourcentages sont exprimés en poids.

## EXEMPLE 1 :

Dans un réacteur de 1 L à double enveloppe muni d'un agitateur, d'un réfrigérant, d'un dispositif de chauffage et d'un bain Huber par la régulation de la température, on place sous agitation 500 g d'IPDI. On chauffe le milieu réactionnel à 60°C. On ajoute 5 mL de bis(triméthylsilyl)amidure de potassium à 0,5 M dans du toluène représentant une quantité de catalyseur de 0,1 %.

Le milieu réactionnel se colore légèrement en jaune.

Il se produit une forte exothermie. La température s'élève jusqu'à 107°C après 7 minutes.

À la fin de l'exothermie, on ajoute 2 mL de dibutylphosphate. Il se produit une décoloration immédiate du milieu réactionnel et un arrêt de la réaction de trimérisation.

Le taux de transformation de l'IPDI est de 68,7%.

**EXEMPLE 2 :**

On répète l'exemple 1 mais en changeant la quantité de catalyseur (0,1 g).
Il ne se produit pas d'exothermie.
On ajoute encore 1 mL de catalyseur après 14,5 heures de réaction. La quantité totale de catalyseur est de 0,04%.
Il se produit alors une légère exothermie. Le milieu réactionnel se teint en jaune.
La réaction est stoppée après 3 heures par addition de 2 mL de dibutylphosphate à 20°C. Le taux de transformation de l'IPDI est de 8,2%.

**EXEMPLE 3 :**

On répète l'exemple 1 en faisant barboter de l'argon dans le milieu réactionnel avant l'addition du catalyseur.
La quantité de catalyseur ajoutée est de 1,6 mL (0,04%).
L'exothermie prend fin 10 minutes après le début de la réaction (température : 75°C).
30 minutes après le début de l'addition du catalyseur, on ajoute 0,17 g de dibutylphosphate (solution à 0,5 M dans le toluène). Il se produit une décoloration importante du milieu réactionnel. Le taux de transformation de l'IPDI est de 51,7%.

**EXEMPLE 4 :**

On procède comme à l'exemple 3, sauf que la quantité d'IPDI est de 400 g et la quantité de catalyseur ajoutée de 0,03% (1,2 ml d'une solution à 0,5 M dans le toluène). La température s'élève à 150°C après 4 minutes.
On ajoute, 30 minutes après la fin de l'exothermie, 0,13 g de dibutylphosphate. Il se produit une décoloration partielle du milieu réactionnel. Le taux de transformation de l'IPDI est de 57,7%.

**EXEMPLE 5 :**

On procède comme à l'exemple 3, sauf que l'on charge 400 g d'IPDI dans le réacteur et on ajoute 1,2 mL de catalyseur (0,03% en poids). La température du milieu réactionnel s'élève à 150°C après 3 minutes. On arrête la réaction 1,5 heures après l'addition du catalyseur par addition de 0,13 g de dibutylphosphate. On observe une décoloration faible du milieu réactionnel.

**EXEMPLE 6 :**

On procède comme à l'exemple 1, sauf que l'on charge le réacteur avec 200g d'IPDI, et on ajoute 6 mL d'une solution 1 M de bis(triméthylsilyl)amidure de lithium dans l'hexane ce qui correspond à 0,5% en poids de catalyseur dans le milieu réactionnel.
La température de la réaction est de 60°C. La réaction est arrêtée après 4 heures par additions de 1,5 g de dibutylphosphate. Le taux de transformation de l'IPDI est de 32,3%.

**EXEMPLE 7 :**

On répète l'exemple 1 avec 200 g d'IPDI et 0,4% de sel de lithium de l'HMDZ (solution 1 M dans l'hexane).
La température de la réaction est de 60°C. La réaction est stoppée après 2 heures par addition de 1 g de dibutylphosphate. On observe une décoloration partielle. Le taux de transformation de l'IPDI est de 29,6%.

**EXEMPLE 8 :**

On procède comme à l'exemple 3 en chargeant le réacteur avec 263,8 g d'HDI et en ajoutant 0,53 mL de sel de potassium de l'HMDZ (solution à 0,5 M dans le toluène). La température du milieu réactionnel monte à 60°C. Après 1 heure de réaction, on chauffe à 90°C. La réaction est stoppée après 3,25 heures par addition de 1 mL de dibutylphosphate.
Le taux de transformation de l'HDI est de 23,6%.
Le spectre I.R. correspond à celui d'un isocyanurate d'HDI (1463 $cm^{-1}$ et 1684 $cm^{-1}$).

**EXEMPLE 9 :**

On répète l'exemple 1 en chargeant le réacteur avec 20 g d'IPDI et en remplaçant le catalyseur par du triméthylsilanolate de lithium (1 g). La température du milieu réactionnel est de 60°C. On stoppe la réaction par addition de dibutylphosphate (0,01 mole).
Le taux de transformation de l'IPDI est de 72%.
Les résultats des essais sont récapitulés dans le tableau ci-dessous.

TABLEAU

| ***Produit / Élément*** | ***Ex. 1*** | ***Ex. 2*** | ***Ex. 3*** | ***Ex. 4*** | ***Ex. 5*** | ***Ex. 6*** | ***Ex. 7*** | ***Ex. 8*** | ***Ex. 9*** |
|---|---|---|---|---|---|---|---|---|---|
| IPDI (%) | 39,3 | 91,5 | 53,0 | 45,4 | 49,1 | 67,8 | 73,7 | - | 28,0 |
| HDI | | | | | | | | 76,4 | |
| Catalyseur(1) | K, HMDZ (0,1) | K, HMDZ (0,04) | K, HMDZ (0,04) | K, HMDZ (0,03) | K, HMDZ (0,03) | Li, HMDZ 0,5) | Li, HMDZ (0,4) | K, HMDZ (0,04) | Li, 8 $(Me)_3$ SiO (5) |
| Température | 60°C | 60°C | 60°C | 120°C | 150°C | 60°C | 60°C | 90°C | 60°C |
| Barbotage argon | Non | Non | Oui | Oui | Oui | Non | Non | Oui | Non |
| Trimère + dimère (%) | 33,1 | 5,6 | 27,7 | 33,9 | 33,8 | 20,1 | 16,4 | - | 37,9 |
| Bis-trimère (%) | 15,9 | 2,1 | 12,4 | 13,7 | 11,8 | 8,6 | 7,0 | - | 20,9 |
| Lourds (tris-trimère) (%) | 11,7 | 0,8 | 6,9 | 7,0 | 5,2 | 3,5 | 2,9 | | 13,2 |
| (1) % en poids par rapport au poids d'isocyanate | | | | | | | | | |

**EXEMPLE 10 :**

Dans un réacteur de 50 mL à double enveloppe muni d'un agitateur, d'un réfrigérant, d'un dispositif de chauffage et d'un bain Huber par la régulations de la température, on place sous agitation 20 g (0,09 moles, 0,18 moles NCO) d'IPDI. On chauffe le milieu réactionnel à 60°C. On ajoute 1 g (PM = 648) de [tris(triméthylsilyl)]amidure d'erbium représentant une quantité de catalyseur de 5 % en poids par rapport au poids de l'IPDI rapporté au métal ($1{,}5.10^{-3}$ moles de catalyseur).
Après cinq heures, on ajoute 300 mg de dibutylphosphate. Il se produit un arrêt de la réaction de trimérisation. Le taux de transformation de l'IPDI est de 23,2%.
Les résultats de l'essai sont récapitulés dans le tableau ci-dessous :

| ***Produit/Élément*** | ***% en poids par rapport au poids d'isocyanate*** |
|---|---|
| IPDI (%) | 76,8 % |
| Triméthylsilylurée d'IPDI | 2,0 % |
| Dimère vrai (mono-urétidione) | Trace |
| Urée ou di-urée non identifiée | |
| Trimère (+ aza-uracile + biuret) | 15,9 % |
| Lourds | 5,4 % |

**EXEMPLE 11 :**

Dans un réacteur de 50 mL à double enveloppe muni d'un agitateur, d'un réfrigérant, d'un dispositif de chauffage

et d'un bain Huber par la régulation de la température, on place sous agitation 20 g (0,09 moles, 0,18 moles NCO) d'IPD1. On chauffe le milieu réactionnel à 60°C. On ajoute 1 g ($2.275.10^{-3}$ moles) de [tris(triméthylsilyl)]amidure d'étain représentant une quantité de catalyseur de 5 % en poids par rapport au poids de l'IPDI de départ.

Après 5 heures de réaction à 60°C, on ajoute 300 mg de dibutylphosphate. Il se produit un arrêt de la réaction.

Le taux de transformation de l'IPDI est de 26,5%.

Les résultats de l'essai sont récapitulés dans le tableau ci-dessous :

| *Produit/Élément* | *% en poids par rapport au poids d'isocyanate* |
|---|---|
| IPDI (%) | 74,5 % |
| Triméthylsilylurée d'IPDI | 4,7 % * |
| IUT** | 16.3% |
| Bis-IUT | 4.3% |
| * : Présence de fonctions carbodi-imide.<br>** : 2-imino-4-oxo-1,3-diazétidine | |

**<u>EXEMPLE 12 :</u> Cyclotrimérisation du monomère HDI (Hexaméthylène diisocyanate)**

<u>Mode opératoire</u>

Après introduction de 10 mg de catalyseur dans un ballon bicol sous boîte à gants, on ajoute le monomère. On n'observe qu'une dissolution partielle du catalyseur. On laisse le mélange réactionnel sous agitation pendant 5 heures à 60°C.

Afin de faciliter l'homogénéisation du milieu réactionnel, on réalise un second essai en dissolvant le catalyseur dans 0,5 mL de toluène avant l'ajout du monomère. Le mélange semble homogène. Sous indication spéciale, la réaction est menée pendant 5 heures à 60°C.

On réalise ensuite une analyse I.R. des mélanges réactionnels après essais.

| **Catalyseur** | ***Pourcentage massique*** | ***Solvant*** | ***Aspect du produit brut*** | ***Bandes trimères observées en cm$^{-1}$*** |
|---|---|---|---|---|
| $Y(N(SiMe_{3)2)3}$ | 1% | non | Pâte blanchâtre et solide opaque | 1690 1464.5 |
| $Y(N(SiMe_3)_2)_3$ | 1% | oui | Pâte blanchâtre | 1686 1466.5 |

L'interprétation des spectres indique que la trimérisation du monomère a bien lieu. On utilise donc ces conditions opératoires. Cependant, on dissout le catalyseur dans le toluène pour conserver une concentration homogène du catalyseur dans le mélange réactionnel.

**<u>EXEMPLE 13</u> : Comparaison entre le complexe d'yttrium et $NaN(SiMe_3)_2$**

On réalise un nouvel essai en remplaçant le complexe de lanthanide par le sel correspondant. On obtient une grande partie insoluble opaque et une pâte liquide blanchâtre. Une analyse I.R. met en évidence la présence de trimère dans les deux fractions mais les bandes caractéristiques du trimère pour la partie soluble sont faibles. Une analyse G.P.C. / I.R.T.F. indique aussi la présence de trimère mais aussi de bis-trimère, de tris- trimère et de composés lourds avec des fonctions isocyanates. Les mélanges réactionnels n'étant pas totalement solubles, une analyse quantitative n'a pu être effectuée. Le sel de silylamidure permet donc la trimérisation mais la trimérisation est beaucoup plus poussée.

**<u>EXEMPLE 14</u> : Étude paramétrique**

On cherche à déterminer l'influence de la nature du centre métallique du catalyseur, de la quantité de catalyseur et de la température du milieu réactionnel sur la trimérisation du monomère. Une analyse G.P.C. couplée I.R.T.F. permet de déterminer la nature des espèces présentes, de les quantifier et de déterminer le taux de transformation global (GI) et du monomère en trimère (T).

Influence de la nature du centre métallique

| *Centre métallique* | *Aspect du M.R.* | *Bandes trimères en $cm^{-1}$* | *Espèces présentes (Analyse G.P.C. / I.R.T.F.)* | | | | |
|---|---|---|---|---|---|---|---|
| Y | Pâte blanchâtre | 1686 1466.5 | lourds | Tris-trimère | Bis-trimère | trimère | HDI |
| Nd | Pâte blanchâtre | 1689.5 1463.5 | lourds | Tris-trimère | Bis-trimère | trimère | HDI |
| Sm | Pâte jaunâtre | 1689 1463.5 | lourds | Tris-trimère | Bis-trimère | trimère | HDI |

Lors de l'analyse G.P.C. / I.R.T.F., une grande partie du mélange réactionnel est resté insoluble dans le dichlorométhane. Il semble donc que l'on ne soit pas au stade de la trimérisation unitaire du monomère comme le témoigne la présence de bis-trimère, de tris-trimère et de composés lourds avec des fonctions isocyanates. Les analyses quantitatives des espèces présentes ne peuvent donc être prises en compte. Une influence du centre métallique ne peut être mise en évidence. Cependant, on peut conclure que tous les complexes des terres rares et notamment des lanthanides permettent une trimérisation du monomère HDI.

**EXEMPLE 15 : Influence de la quantité de catalyseur**

L'étude a été effectuée sur le catalyseur $Y[N(SiMe_3)_2]_3$.

| *Catalyseur pourcentage massique* | *Aspect du M.R.* | *Bandes trimères en $cm^{-1}$* | *Espèces présentes et leurs quantités (Analyse G.P.C./ I.L.R.T.F.)* | | | | | *T.T.* |
|---|---|---|---|---|---|---|---|---|
| 1% | Pâte blanchâtre | 1686 1466,5 | lourds | Tris-trimère | Bis-trimère | trimère | HDI | |
| 0,2% | Liquide visqueux jaunâtre | 1686,5 Lourds 1465 | 1,2% | Tris-trimère 0,9% | Bis-trimère 2,9% | Trimère+ biuret 12,3% | HDI 40,5% | GI:31% T+B:21% |
| 0,1% | Liquide visqueux jaunâtre | 1686 1460 | | | Bis-trimère 0,7% | Trimère+ biuret 6,3% | HDI 83,3% | GI:8% T+B:7% |

En diminuant la quantité de catalyseur, la réaction est moins poussée.

**EXEMPLE 16 : Influence de la température**

Les essais sont effectués sur des mélanges réactionnels contenant 0,1% en rapport massique de catalyseur $Y[N(SiMe_3)_2]_3$.

| *T en°C* | *Aspect du M.R.* | *Bandes trimères en $cm^{-1}$* | *Espèces présentes et leurs quantités (Analyse G.P.C./ I.R.T.F.)* | | | | | *T.T.* |
|---|---|---|---|---|---|---|---|---|
| 60 | Liquide visqueux jaunâtre | 1686,5 1465 | | | Bis-trimère 0,7% | Trimère +biuret 6,3% | HDI 83,3% | GI:8% T+B: 7% |
| 100 | Liquide visqueux jaunâtre | 1689 1464,5 | lourds 0,8% | Tris-trimère | Bis-trimère 0,4% 2,1% | Trimère 13% | HDI 64,3% | GI:25% T: 20% |

**TRIMÉRISATION À PARTIR DE BENZIMIDINATE OU "SIAM"** (exemples 17, 18)

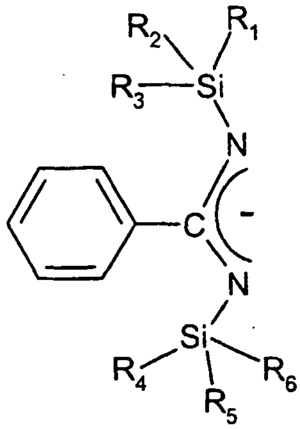

**EXEMPLE 17 : Comparaison entre le Samarium et Na**

Les manipulations ont été réalisées selon le même mode opératoire que précédemment.

| *Catalyseur* | *Pourcentage massique* | *Aspect du produit brut* | *Bandes trimères observées en $cm^{-1}$* |
|---|---|---|---|
| $Sm(Siam)_3PhCN$ | 1% | Pâte opaque | 1688.5 1464 |
| NaSiam | 1% | Solide opaque | 1686 1465.5 |
| LiSiam | 1% | Solide opaque | 1689 1465 |

Les 3 mélanges réactionnels présentant des insolubles, l'analyse G.P.C. / I.R.T.F. n'a pu permettre uniquement de qualifier les espèces présentes. Elle indique la présence de trimère, de bis-trimère, de tris-trimère et de composés lourds à fonctions isocyanates présentant majoritairement au moins un cycle isocyanurique. Le stade de trimérisation est trop avancé pour pouvoir réaliser des mesures significatives, la catalyse est cependant très forte.

**Étude de l'influence des paramètres expérimentaux**

**Influence de la nature du centre métallique**

| *Centre métallique* | *Aspect du M.R.* | *Bandes trimères en $cm^{-1}$* | *Espèces présentes (Analyse G.P.C./I.R.T.F.)* | | | | |
|---|---|---|---|---|---|---|---|
| Sm | Pâte opaque | 1688.5 1464 | lourds | Tris-trimère | Bis-trimère | trimère | HDI |
| Yb | Pâte opaque | 1688.5 1463 | lourds | Tris-trimère | Bis-trimère | trimère | HDI |
| Y | Pâte blanchâtre | 1688.5 1463 | lourds | Tris-trimère | Bis-trimère | trimère | HDI |

La présence d'insolubles n'a pas permis de quantifier les produits bruts obtenus. Tous les sels de terre rare constituent des catalyseurs puissants de trimérisation.

**EXEMPLE 18 : Influence de la quantité de catalyseur (métal yttrium)**

| ***Teneur en % masse*** | ***Aspect du M.R.*** | ***Bandes trimères en cm$^{-1}$*** | ***Espèces présentes et leurs quantités (Analyse G.P.C./ I.R.T.F.)*** | | | | | ***T.T.*** |
|---|---|---|---|---|---|---|---|---|
| 1% | Pâte blanchâtre | 1688.5 1463 | lourds | Tris-trimère | Bis-trimère | trimère | HDI | |
| 0,2% | Liquide visqueux, jaunâtre 1,7% 4,3% 13,4% | 1686 1460 | lourds 2,7% | Tris-trimère | Bis-trimère | Trimère +biuret | HDI 41,3% | GI:35% T+B:21% |

La diminution de la quantité de catalyseur a entraîné la disparition des insolubles et a permis de diminuer le stade de trimérisation.

**Cyclotrimérisation du monomère IPDI (isophorone diisocyanate)**

**EXEMPLE 19 : Comparaison entre le sel d'Yttrium et $NaN(SiMe_3)_2$**

Les essais sont réalisés dans les mêmes conditions opératoires que pour le monomère HDI (cf. EXEMPLE 12).

| ***Catalyseur*** | ***Aspect du M.R.*** | ***Bandes trimères en cm$^{-1}$*** | ***Espèces présentes et leurs quantités (Analyse G.P.C./I.R.T.F.)*** | | | | |
|---|---|---|---|---|---|---|---|
| $Y(N(SiMe_3)_2)_3$ (1% en masse) | liquide jaunâtre | Peu importante | lourds | Biuret+ trimère | Urée | Carbamate | IPDI |
| $NaN(SiMe_3)_2$ (1% en masse) | Pâte jaunâtre | 1692 1446.5 | lourds | Tris-trimère | Bis-trimère | Trimère | IPDI |

Comme prévu, le rendement dépend du cation associé. L'efficacité du sel alcalin est beaucoup plus forte que celle des sels d'yttrium.

Dans la perspective d'une application industrielle, la durée de réaction et/ou la quantité de catalyseur devra être diminuée pour les alcalins cependant que les conditions opératoires devront être durcies pour les sels d'yttrium.

**Étude de l'influence de paramètres expérimentaux**

La trimérisation de l'IPDI dans les conditions précédant ne donnant pas de résultats satisfaisants, on va donc modifier les paramètres opératoires.

**EXEMPLE 20 : Influence de la nature du cation associé (même anion que dans l'exemple 19)**

| *Centre métallique* | *Aspect du M.R. du* | *Bandes trimères en $cm^{-1}$* | *Espèces présentes et leurs quantités (Analyse G.P.C./ I.R.T.F.)* | | | | *T.T.* |
|---|---|---|---|---|---|---|---|
| Y | Liquide peu visqueux jaunâtre | faible | Trimère + biuret | Urée | Carbamate | IPDI | |
| Sm | Liquide peu visqueux jaunâtre | faible | Trimère + biuret 1,5 | Urée 4,3% | Dimère 0,4% | IPDI 76% | GI: 0,1% |

Les conditions opératoires sont trop douces pour ces cations associés.
Le samarium donne des résultats un peu meilleurs et une formation de dimère.
Le néodyme donne des résultats similaires à ceux de l'yttrium.
Les lanthanides semblent plus légèrement efficaces que les autres terres rares (Y, Sc)

**EXEMPLE 21 : Influence de la température**

L'étude de ce paramètre a été effectuée sur le sel d'HMDZ d'yttrium à 1%.

| *T en °C* | *Aspect du M.R. du* | *Bandes trimères en $cm^{-1}$* | *Espèces présentes et leurs quantités (Analyse G.P.C./ I.R.T.F.)* | | | | *T.T.* |
|---|---|---|---|---|---|---|---|
| 60 | Liquide peu visqueux jaunâtre | Faible | Biuret + trimère | Urée | Carbamate | IPDI | |
| 100 | Liquide peu visqueux jaunâtre | oui | Biuret + trimère 2,2% | Urée 5% | Carbamate | IPDI 53% | GI:14% T+B: 4% |

Une hausse de température semble donc favoriser la condensation du monomère IPDI. Un essai à 5% en catalyseur à la même température (100°C) a permis d'augmenter la condensation et d'obtenir de meilleurs taux de transformation (GI : 26% et T : 16%).

**Trimérisation à partir des complexes benzimidinate (siam)**

**EXEMPLE 22 : Comparaison entre les sels de complexe de samarium et Na-siam**

| *Catalyseur* | *Pourcentage massique* | *Aspect du produit brut* | *Bandes trimères observées en $cm^{-1}$* |
|---|---|---|---|
| $Sm(Siam)_3PhCN$ | 1% | Liquide peu visqueux jaune | Non significative |
| NaSiam | 1% | Pâte jaune | 1690 1445 |

Un lavage au pentane ayant été effectué, l'analyse G.P.C. / I.R.T.F. montre la présence de trimère, de bis-trimère, de tris-trimère et de composés lourds à fonctions isocyanates pour le sel. Pour le sel de samarium, la présence de trimère n'a pu être mis en évidence; en revanche, on trouve de faible quantité de biuret et des composés condensés.

**EXEMPLE 23 : Étude de l'influence des paramètres expérimentaux Influence de la quantité de catalyseur ($Sm(siam)_3PhCN$)**

| Pourcentage massique | Aspect du M.R. | Espèces présentes (Analyse G.P.C./I.R.T.F.) | | | |
|---|---|---|---|---|---|
| 1% | Liquide peu visqueux jaune | Biuret | Urée | Carbamate | IPDI |
| 5% | Liquide peu visqueux jaunâtre | Biuret + trimère | Urée | Carbamate | IPDI |

L'augmentation de catalyseur permet de mieux mettre en évidence la formation de trimère et d'autres condensations.

Une augmentation de la température permet l'obtention de rendement plus important

Ainsi un essai a été effectué à 100°C avec un sel d'yttrium à 5%. Les analyses montrent la présence de trimère et de biuret avec les taux de transformations suivants : GI : 16.5% et T + B : 9%.

## Revendications

1. Utilisation de composé(s) salins(s) comme catalyseur de (cyclo)condensation de fonction isocyanate avantageusement aliphatique, y compris cycloaliphatique, **caractérisée par le fait que** ledit composé salin est choisi parmi ceux qui répondent à la formule :

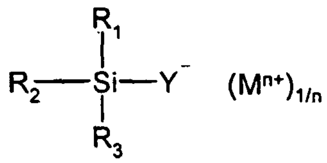

où $(M^{n+})_{1/n}$ représente le(s) co-cation(s) nécessaire(s) à la neutralité électrique dudit composé salin équilibrant l'anion de formule (I) :

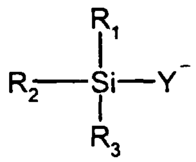

(I)

où $Y^-$ est choisi parmi :

- un oxygène chargé négativement ;
- et un radical carboné comportant une charge négative portée par un atome de la colonne VB ou par un oxygène, avantageusement par un azote et dont la liaison avec le silicium de la formule (I) ci-dessus est portée par un atome de la colonne VB, avantageusement par un azote ;

où $R_1$, $R_2$ et $R_3$, identiques ou différents, représentent un groupe monovalent hydrocarboné ayant avantageusement de 1 à 30 atomes de carbone, éventuellement substitué par un ou plusieurs atomes d'halogène, groupements CN, ou ester, et/ou éventuellement interrompu par un ou plusieurs atomes choisis parmi O, N et Si, avantageusement de nature aliphatique, y compris cycloaliphatique saturé ou insaturé et aralcoyle, ou de nature aromatique tel que aryle ou alcoylaryle ; et

parmi les sels dont le(s) composé(s) anionique(s) (porteurs d'une charge négative) sont porteurs d'un groupe trialcoylsilyle et résulte(nt) de la réaction desdits composés de formule (I) et d'une fonction isocyanate avantageusement aliphatique.

2. Utilisation selon la revendication 1, **caractérisée par le fait que** Y représente un radical de formule (II) :

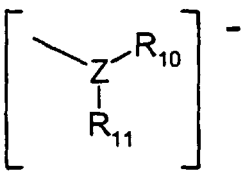

(II)

dans laquelle :

- Z représente un métalloïde de la colonne VB ;
- $R_{10}$ est choisi parmi les radicaux comportant de 1 à 30 atomes de carbone, y compris silyle et notamment trialcoylsilyle ; et
- $R_{11}$ est choisi parmi une charge négative (anionique) et un groupe carboné (avantageusement d'au plus 30 atomes de carbone) attaché à Z par un carbone à la fois d'hybridation $sp^2$ et porteur d'un azote dont l'azote porte une charge négative (anionique) ;

**3.** Utilisation selon la revendication 1, dans laquelle l'un au moins des $R_1$, $R_2$, et $R_3$, est interrompu par plusieurs atomes choisis parmi O, N et Si, formant la séquence (I') :

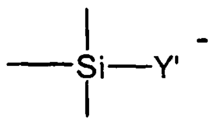

dans laquelle $Y'^-$ représente un oxygène chargé négativement ou une séquence (II') :

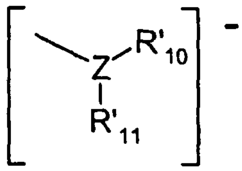

dans laquelle

- Z' représente un métalloïde de la colonne VB ;
- $R'_{10}$, représente un groupe hydrocarboné relié au radical Z' par un carbone ou un silicium ; (cf. radical $R_{10}$) ;
- $R'_{11}$ est la charge négative de Y' ou une séquence sur un atome d'azote de Y' comportant un carbone $sp^2$ à la fois lié à Z' et porteur d'un azote porteur de la charge négative.

**4.** Utilisation selon les revendications 2 et 3, **caractérisée par le fait que** lorsque $R_{11}$ représente une charge négative, $R_{10}$ présente avantageusement d'au plus 30 atomes de carbone et est choisi parmi les radicaux hydrocarbonés, notamment les aryles, les alcoyles et les silyles, et parmi les groupes carbonés attachés à Z par un carbone, lequel est à la fois d'hybridation $sp^2$ et porteur d'un azote.

**5.** Utilisation selon les revendications 2 à 4, **caractérisée par le fait que** lorsque $R_{11}$, représente une charge négative, $R_{10}$ présente avantageusement d'au plus 30 atomes de carbone et est choisi parmi les radicaux alcoyles, silyles, et les groupes carbonés attachés à Z par le carbone d'hybridation $sp^2$ une séquence iminométhylènyle

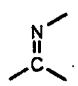

ou d'une séquence -C O-N<

6. Utilisation selon les revendications 4 et 5, **caractérisé par le fait que** $R_{10}$ est de formule :

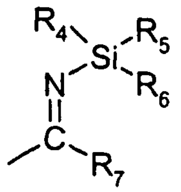

7. Utilisation selon les revendications 2 à 6, **caractérisée par le fait que** $R_{10}$ est de formule :

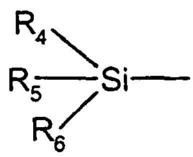

et $R_{11}$ représente une charge négative ou est de formule:

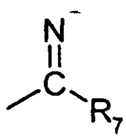

dans laquelle :

- $R_4$, $R_5$ et $R_6$ identiques ou différents représentent un groupe monovalent de nature hydrocarbonée ayant de 1 à 30 atomes de carbone, aliphatique, cycloaliphatique saturé ou insaturé, aryle, aralcoyle ou alcoylaryle, éventuellement substitué par un ou plusieurs atomes d'halogène, groupements CN, ou ester, et/ou éventuellement interrompu par un ou plusieurs atomes choisis parmi O, S, N et Si ;
- $R_7$, représente avantageusement un radical hydrocarboné d'au plus 30 carbones, avantageusement de nature aliphatique (c'est-à-dire dont le carbone porteur du lien avec l'imine est d'hybridation $sp^3$) notamment alcoyle, ou avantageusement un aryle de préférence isocyclique.

8. Utilisation selon les revendications 1 à 7, **caractérisée par le fait que** le composé de formule (I) répond à l'une des formules (Va) ou (Vb) suivantes :

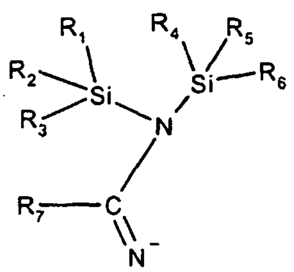

;

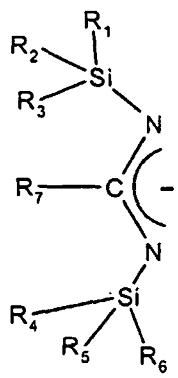

(Va) (Vb)

**9.** Utilisation selon les revendications1 à 7, **caractérisé par le fait que** ledit composé de formule (I) est choisi parmi les composés de formules (1) et (2) :

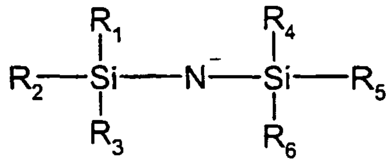

(1)

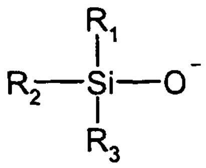

(2)

où $R_1$, $R_2$, $R_3$, $R_4$, $R_5$ et $R_6$, identiques ou différents, représentent un groupe monovalent de nature hydrocarbonée ayant de 1 à 30 atomes de carbone, aliphatique, cycloaliphatique saturé ou insaturé, aryle, aralcoyle ou alcoylaryle, éventuellement substitué par un ou plusieurs atomes d'halogène, groupements CN, ou ester, et/ou éventuellement interrompu par un ou plusieurs atomes choisis parmi O, S, N et Si.

**10.** Utilisation selon les revendications 1 à 9, **caractérisée par le fait que** lesdits co-cations sont choisis parmi les oniums et les cations métalliques n'ayant avantageusement qu'un seul état d'oxydation stable
ou à leur état d'oxydation le plus bas (état élémentaire excepté).

**11.** Utilisation selon les revendications 1 à 9, **caractérisée par le fait que** lesdits co-cations sont choisis parmi les cations métalliques alcalins et les oniums.

**12.** Utilisation selon les revendications 1 à 9, **caractérisée par le fait que** lesdits co-cations sont choisis parmi les cations métalliques des métaux de transition.

**13.** Utilisation selon les revendications 1 à 10 et 12, **caractérisée par le fait que** lesdits co-cations sont choisis parmi les cations des terres rares.

**14.** Utilisation selon les revendications 12 et 13, **caractérisée par le fait que** lesdits co-cations sont choisis parmi les cations du lanthane et des lanthanides.

**15.** Utilisation selon l'une des revendications 12 à 14,
**caractérisée par le fait que** lesdits co-cations sont les cations de l'erbium.

**16.** Utilisation selon l'une des revendications 1 à 10, **caractérisée par le fait que** lesdits co-cations sont le cation d'étain (II).

**17.** Procédé de (cyclo)condensation, notamment (cyclo)trimérisation catalytique d'isocyanates monomères, **caractérisé en ce qu'**il comprend :

a) la réaction des isocyanates monomères de départ avec un catalyseur comprenant un sel minéral ou organique d'un composé de formule (I) ;
b) à une température d'au moins 20°C et avantageusement d'au moins 40°C et d'au plus 200°C, avantageusement d'au plus 150°C,
c) l'arrêt de la réaction de (cyclo)condensation au taux de transformation souhaité compris avantageusement entre 5 et 95%, de préférence entre 10 et 50%; et
d) l'élimination éventuelle des monomères n'ayant pas réagi ou la réaction avec d'autres composés réactifs avec la fonction isocyanate.

**18.** Procédé de (cyclo)condensation, selon la revendication 17 notamment (cyclo)trimérisation catalytique d'isocyanates monomères, **caractérisé par le fait que** le composé anionique de formule (I) est choisi parmi les composés répondant à l'une des formules ci-après :

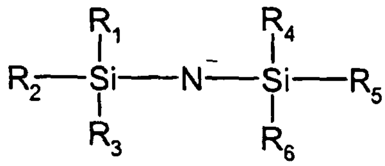

(1)

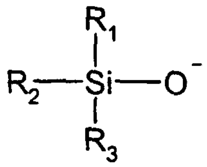

(2)

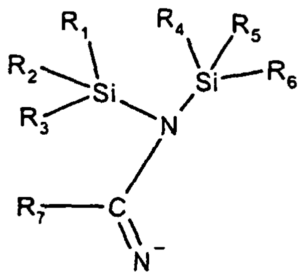

;

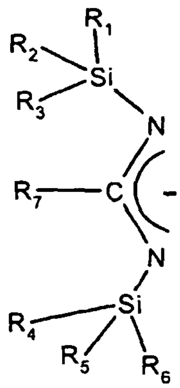

(Va) (Vb)

dans lesquelles $R_1$, $R_2$, $R_3$, $R_4$, $R_5$, $R_6$ et $R_7$, identiques ou différents, sont tels que définis dans les revendications précédentes.

**19.** Procédé de (cyclo)condensation, selon les revendications 17 et 18, notamment (cyclo)trimérisation catalytique d'isbcyanates monomères, **caractérisé par le fait que** le composé anionique de formule (I) est choisi parmi les composés ci-après :

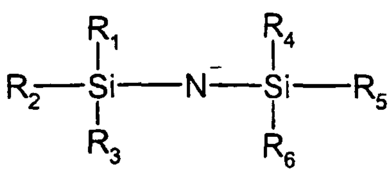

(1)

- avec $R_1$, $R_2$, $R_3$, $R_4$, $R_5$ et $R_6$ identiques ou différents représentent un groupe monovalent de nature hydrocarbonée ayant de 1 à 30 atomes de carbone, aliphatique, cycloaliphatique saturé ou insaturé, aryle, aralcoyle ou alcoylaryle, éventuellement substitué par un ou plusieurs atomes d'halogène, groupements CN, ou ester, et/ou éventuellement interrompu par un ou plusieurs atomes choisis parmi O, S, N et Si
- l'un de $R_1$, $R_2$, $R_3$, $R_4$, $R_5$ et $R_6$ représente un motif de formule :

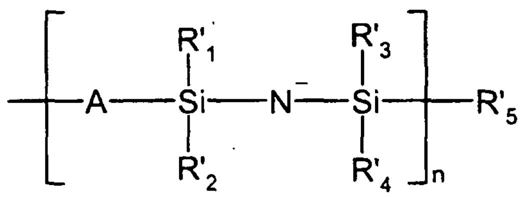

dans laquelle A représente une chaîne alcoylène ayant de 1 à 30 atomes, avantageusement de 2 à 20 atomes de carbone éventuellement substituée par un ou plusieurs atomes d'halogène, groupements CN, ou ester, et/ou éventuellement interrompu par un ou plusieurs atomes choisis parmi O, S, N et Si, de préférence $(CH_2)_{n'}$ avec n' compris entre 1 et 6, avantageusement de 2 à 6,
et $R'_1$ à $R'_5$ identiques ou différents représentant un groupe monovalent de nature hydrocarbonée, aliphatique, cycloaliphatique saturé ou insaturé, aryle, aralcoyle ou alcoylaryle, éventuellement substitué par un ou plusieurs atomes d'halogène, groupements CN, ou ester, et/ou éventuellement interrompu par un ou plusieurs atomes choisis parmi O, S, N et Si, et
n est un nombre entier entre 1 et 50, ou

- deux parmi $R_1$, $R_2$ et $R_3$ d'une part et/ou $R_4$, $R_5$ et $R_6$ d'autre part, constituent ensemble un groupe hydrocarboné divalent, éventuellement substitué par un ou plusieurs atomes d'halogène, groupements CN, ou ester, et/ou éventuellement interrompu par un ou plusieurs atomes choisis parmi O, S, N et Si, et/ou
- au moins un groupe choisi parmi $R_1$, $R_2$ et $R_3$ forme avec au moins un groupe parmi $R_4$, $R_5$ et $R_6$ un groupe hydrocarboné divalent, éventuellement substitué par un ou plusieurs atomes d'halogène, groupements CN, ou ester, et/ou éventuellement interrompu par un ou plusieurs atomes choisis parmi O, S, N et Si, ou un précurseur dudit composé,

**20.** Procédé selon les revendications 17 à 19, **caractérisé en ce que** $R_1$ à $R_6$ et $R'_1$ à $R'_5$ identiques ou différents représentent :

- un groupe alcoyle, alcényle, halogénoalcoyle ou halogénoalcényle ayant de 1 à 20, de préférence de 1 à 6 atomes de carbone, éventuellement substitué par des atomes de chlore et/ou de fluor, et/ou éventuellement interrompu par un ou plusieurs atomes d'azote et/ou de silicium,
- un groupe cycloalcoyle, cycloalcényle, halogénocycloalcoyle

ou halogénocycloalcényle ayant de 3 à 30, de préférence 3 à 10 atomes de carbone et contenant des atomes de chlore et/ou de fluor, et/ou éventuellement interrompu par un ou plusieurs atomes d'azote et/ou de silicium,

- un groupe aryle, alcoylaryle ou halogénoaryle ayant de 6 à 30, de préférence 6 à 10 atomes de carbone et contenant des atomes de chlore et/ou de fluor,
- un groupe cyanoalcoyle ayant de 1 à 6 atomes de carbone,
- ou deux groupes parmi $R_1$, $R_2$ et $R_3$ ou $R_3$, $R_4$ et $R_5$ forment ensemble un radical divalent comprenant de 2 à 5 atomes de carbone, et/ou éventuellement interrompu par un ou plusieurs atomes d'azote et/ou de silicium,
- ou deux parmi $R_1$, $R_2$ et $R_3$ d'une part et/ou $R_4$, $R_5$ et $R_6$ d'autre part, constituent ensemble un groupe hydrocarboné divalent et/ou éventuellement interrompu par un ou plusieurs atomes d'azote et/ou de silicium, ou
- au moins un groupe choisi parmi $R_1$, $R_2$ et $R_3$ forme avec au moins un groupe parmi $R_4$, $R_5$ et $R_6$ un groupe hydrocarboné divalent, comprenant de 2 à 5 atomes de carbone, et/ou éventuellement interrompu par un ou plusieurs atomes d'azote et/ou de silicium.

**21.** Procédé selon les revendications 17 à 20, **caractérisé en ce que** $R_1$ à $R_6$ identiques ou différents sont choisis parmi les groupes méthyle, éthyle, propyle, isopropyle, butyle, isobutyle, $\alpha$-pentyle, t-butyle, chlorométhyle, dichlorométhyle, $\alpha$-chloro-éthyle, $\alpha,\beta$-dichloro-éthyle, fluorométhyle, difluorométhyle, $\alpha,\beta$-difluoro-éthyle, trifluoro-3,3,3-propyle, trifluorocyclopropyle, trifluoro-4,4,4-butyle, heptafluoro-3,3,3,4,4,5,5-pentyle, $\beta$-cyano-éthyle, $\gamma$-cyanopropyle, phényle, p-chlorophényle, m-chlorophényle, dichloro-3,5 phényle, trichlorophényle, tétrachlorophényle, o-, p-, ou m-tolyle, $\alpha,\alpha,\alpha$-trifluorotolyle, et xylyles ;
ou deux groupes parmi $R_1$, $R_2$ et $R_3$ ou $R_4$, $R_5$ et $R_6$ représentent un groupe diméthylène, triméthylène, tétraméthylène ou pentaméthylène et/ou $R_1$ et $R_4$ pris ensemble représentent un groupe choisi parmi éthylène, propylène, butylène, pentylène, de préférence les isomères non ramifiés, de méthylène et triméthylène.

**22.** Procédé selon les revendications 17 à 21, **caractérisé en ce que** $R_1$ à $R_6$ et $R'_1$ $R'_5$ identiques ou différents sont choisis parmi méthyle, éthyle, propyle, vinyle et phényle, ces groupes étant éventuellement chlorés et/ou fluorés.

**23.** Procédé selon l'une quelconque des revendications 17 à 22,
**caractérisé en ce que** le composé de formule (1) est choisi parmi les sels des composés suivants :

hexaméthyldisilazane,
diéthyl-1,3-tétraméthyl-1,1,3,3-disilazane,
divinyl-1,3-tétraméthyl-1,1,3,3-disilazane,
hexaéthyldisilazane,
diphényl-1,3-tétraméthyl-1,1,3,3-disilazane,
hexaméthylcyclotrisilazane, et
éthylène-2,5-tétraméthyl-2,2,5,5-cyclodisilazane.

**24.** Procédé selon la revendication 18, **caractérisé en ce que** le composé de formule générale (2) est choisi parmi :

le triméthylsilanolate,
le triéthylsilanolate,
l'éthyldiméthylsilanolate, et
le vinyldiméthylsilanolate.

**25.** Procédé selon l'une quelconque des revendications 17 à 24, **caractérisé en ce que** M représente K, Li, Na ou Mg.

**26.** Procédé selon l'une quelconque des revendications 17 à 24, **caractérisé en ce** le sel du composé de formule (I) est un sel d'erbium du composé de formule (1), et répond à la formule générale ($1^E$) suivante, lorsque n' représente zéro :

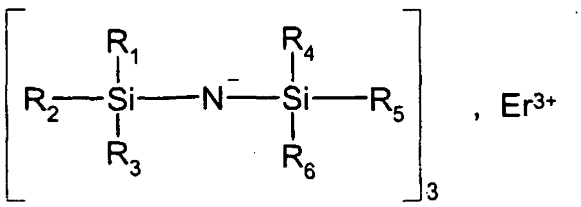

($1^E$)

**27.** Procédé selon la revendication 26, **caractérisé en ce que** le sel d'erbium est choisi parmi les sels d'erbium de l'hexaméthyldisilazane, du diéthyl-1,3-tétraméthyl-1,1,3,3-disilazane, du divinyl-1,3-tétraméthyl-1,1,3,3-disilazane, de l'hexa-éthyldisilazane, et du diphényl-1,3-tétraméthyl-1,1,3,3-disilazane.

**28.** Procédé selon l'une quelconque des revendications 17 à 24, **caractérisé en ce** le sel du composé de formule (I) est un sel d'étain (II) du composé de formule (1), et répond à la formule générale ($1^S$) suivante, lorsque n' représente zéro :

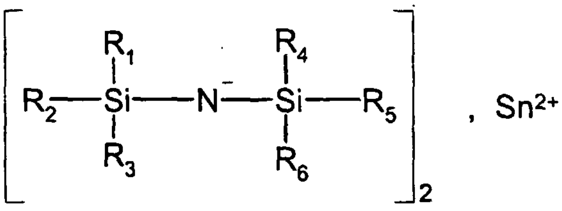

(1

**29.** Procédé selon la revendication 28, **caractérisé en ce que** le sel d'étain (II) est choisi parmi les sels d'étain de l'hexaméthyldisilazane, du diéthyl-1,3-tétraméthyl-1,1,3,3-disilazane, du divinyl-1,3-tétraméthyl-1,1,3,3-disilazane, de l'hexa-éthyldisilazane, du diphényl-1,3-tétraméthyl-1,1,3,3-disilazane, de l'hexaméthylcyclotrisilazane et de

l'éthylène-2,5-tétraméthyl 2,2,5,5-cyclodisilazane.

**30.** Procédé selon l'une quelconque des revendications 17 à 29, **caractérisé en ce que** l'isocyanate de départ est un diisocyanate dans lequel au moins une, avantageusement les deux fonctions isocyanates sont portées par un atome de carbone en position cycloaliphatique, secondaire, tertiaire ou néopentylique.

**31.** Procédé selon l'une quelconque des revendications 17 à 30,
**caractérisé en ce que** ledit isocyanate est un isocyanate cycloaliphatique dans lequel au moins une, avantageusement les deux fonctions isocyanates est distante du cycle le plus proche d'au plus un atome de carbone, de préférence reliée directement à lui.

**32.** Procédé selon la revendication 31, **caractérisé en ce que** l'isocyanate est l'IPDI.

**33.** Procédé selon l'une quelconque des revendications 17 à 32, **caractérisé en ce que** le ratio molaire quantité de catalyseur / quantité de fonctions NCO est d'au moins $5.10^{-5}$, et d'au plus $5.10^{-3}$.

**34.** Composition isocyanate susceptible d'être obtenue par le procédé défini dans l'une quelconque des revendications 17 à 33.

**35.** Composition isocyanate **caractérisée par le fait qu'**elle comporte des composés porteurs de fonctions isocyanates, avantageusement aliphatiques, et un composé salin choisi parmi ceux répondant à la formule :

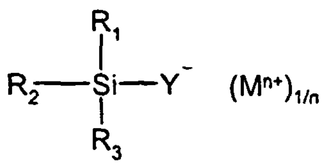

où $(M^{n+})_{1/n}$ est le(s) co-cation(s) nécessaire(s) à la neutralité électrique dudit composé salin équilibrant l'anion de formule (I) :

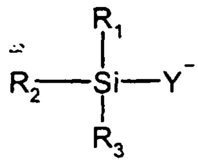

(I)

où $Y^-$ est choisi parmi:

- un oxygène chargé négativement ;
- ou un radical carboné comportant une charge négative portée par un atome de la colonne VB ou par un oxygène avantageusement par un azote et dont la liaison avec le silicium de la formule (I) ci-dessus est portée par un atome de la colonne VB, avantageusement par un azote ;

où $R_1$, $R_2$ et $R_3$, identiques ou différents, représentent un groupe monovalent hydrocarbonée ayant avantageusement de 1 à 30 atomes de carbone, éventuellement substitué par un ou plusieurs atomes d'halogène, groupements CN, ou ester, et/ou éventuellement interrompu par un ou plusieurs atomes choisis parmi O, N et Si, avantageusement de nature aliphatique, y compris cycloaliphatique saturé ou insaturé et aralcoyle, ou de nature aromatique tel que aryle ou alcoylaryle ;
et
parmi les sels dont le(s) composé(s) anionique(s) (porteurs d'une charge négative) sont porteurs d'un groupe trialcoylsilyle et résulte(nt) de la réaction desdits composés de formule (I) et d'une fonction isocyanate avantageusement aliphatique le ratio molaire quantité de catalyseur / quantité de fonctions NCO est d'au moins $5.10^{-5}$, et d'au plus $5.10^{-2}$.

**36.** Composition isocyanate selon l'une quelconque des revendications 34 et 35, **caractérisée par le fait que** la concentration en fonction isocyanate libre est comprise entre 0,5 et 7 équivalents par litre.

**37.** Composition isocyanate selon l'une quelconque des revendications 34 à 36, **caractérisée par le fait qu'**elle comporte en masse au moins 1%, avantageusement au moins 3%, de préférence au moins 5% de composé présentant au moins un motif :

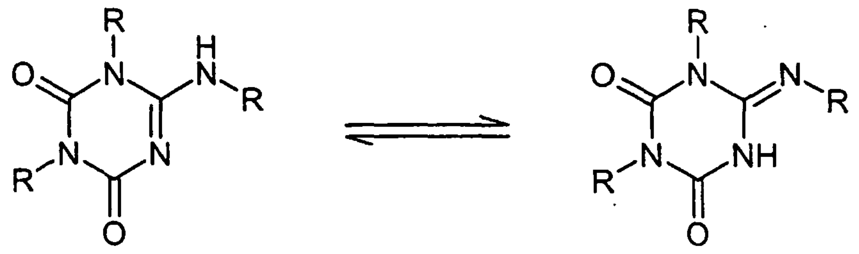

où les R, identiques ou différents, représentent les restes du, ou des monomères, après ignorance d'une des fonctions isocyanates, les autres pouvant être engagées dans une condensation distincte.

**38.** Composition isocyanate selon l'une quelconque des revendications 34 à 36, **caractérisée par le fait qu'**elle comporte en masse au moins 1%, avantageusement au moins 3%, de préférence au moins 5% de composé présentant au moins un motif :

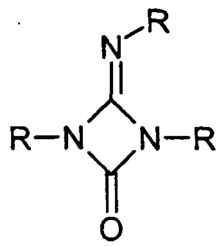

où les R, identiques ou différents, représentent les restes du, ou des monomères, après ignorance d'une des fonctions isocyanates, les autres pouvant être engagées dans une condensation distincte.

**39.** Composition isocyanate selon l'une quelconque des revendications 34 à 36, **caractérisée en ce qu'**elle a été mise en oeuvre avec des sels d'erbium, et **caractérisée en ce qu'**elle comprend de 50 à 80% en poids de composés trimères consistant en mono-isocyanates et 6-amino-5-aza-uraciles, de 0,5 à 5%, avantageusement de 0,5 à 3% de composés mono-urétidiones, et moins de 1% en poids d'isocyanates monomères, par rapport au poids total des composés isocyanates présents dans la composition.

**40.** Composition isocyanate selon l'une quelconque des revendications 34 à 36, **caractérisée en ce qu'**elle a été mise en oeuvre avec des sels d'étain, et **caractérisée en ce qu'**elle comprend de 50 à 75%, avantageusement de 55 à 70% en poids de composés mono-2-imino-4-oxo-1,3-diazétidine, de 10 à 25 % en poids, avantageusement de 12 à 20% en poids de composés bis-(2-imino-4-oxo-1,3-diazétidine), de 10 à 25%, avantageusement de 14 à 20% en poids d'isocyanates-urées, et moins de 1 % en poids d'isocyanates monomères, par rapport au poids total des composés isocyanates présents dans la composition.

**41.** Composition isocyanate selon l'une quelconque des revendications 34 à 40, **caractérisée en ce qu'**elle comprend moins de 1 % en poids d'isocyanates monomères par rapport au poids total des composés isocyanates présents dans la composition, ladite composition comprenant de 0,5 à 100 %, avantageusement de 10 à 100 %, de préférence de 20 à 100 % de fonctions isocyanates masquées à l'aide d'un agent masquant de la fonction isocyanate.

**42.** Utilisation des compositions isocyanates selon l'une quelconque des revendications 34 à 41 dans l'élaboration de produits de revêtement, peintures et vernis, d'adhésifs, d'encres, de biocides, de détergents.

## Claims

1. The use of salt compound(s) as a (cyclo)condensation catalyst with isocyanate function, advantageously aliphatic, including cycloaliphatic,
**characterised by** the fact that said salt compound is chosen from those which correspond to the formula:

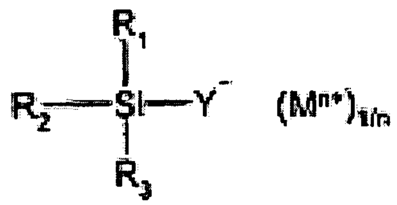

where $(M^{n+})_{1/n}$ represents the cocation(s) required for the electric neutrality of said salt compound balancing the anion with formula (I):

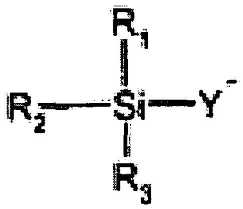

(I)

where Y is chosen from:

. a negatively charged oxygen;
. and a carbonaceous radial comprising a negative charge carried by an atom from column VB or by an oxygen, advantageously by a nitrogen, and the binding of which with the silicon with the above formula (I) is carried by an atom from column VB, advantageously by a nitrogen;

- where $R_1$, $R_2$ and $R_3$, identical or different, represent a monovalent hydrocarbonaceous group advantageously having 1 to 30 carbon atoms, possibly substituted by one or more halogen atoms, CN groups, or ester, and/or possibly interrupted by one or more atoms chosen from O, N and Si, advantageously aliphatic in nature, including saturated or unsaturated cycloaliphatic and aralkyl, or aromatic in nature such as aryl or alkylaryl;
and
from the salts the anionic compound(s) of which (carriers of a negative charge) is/are carriers of a trialkylsilyl group and result(s) from the reaction of said compounds with formula (I) and an isocyanate function, advantageously aliphatic.

2. The use according to Claim 1, **characterised by** the fact that Y represents a radical with formula (II):

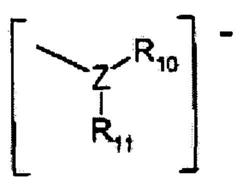

(II)

wherein:

. Z represents a metalloid from column VB;
. $R_{10}$ is chosen from the radicals comprising 1 to 30 carbon atoms, including silyl and in particular trialkylsilyl; and

. $R_{11}$ is chosen from a negative (anionic) charge and a carbonaceous group (advantageously of at most 30 carbon atoms) attached to Z by a carbon which is both of $sp^2$ hybridization and the carrier of a nitrogen, the nitrogen of which carries a negative (anionic) charge);

**3.** The use according to Claim 1, wherein at least one of $R_1$, $R_2$ and $R_3$ is interrupted by a number of atoms chosen from O, N and Si forming the sequence (I'):

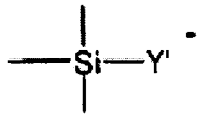

wherein $Y'^-$ represents a negatively charged oxygen or a sequence (II'):

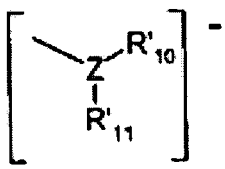

wherein

. Z' represents a metalloid from column VB;
. $R'_{10}$ represents a hydrocarbonaceous group linked to the Z' radical by a carbon or a silicon; (see radical $R_{10}$);
. $R'_{11}$ is the negative charge of Y' or a sequence on a nitrogen atom of Y' comprising a $sp^2$ carbon both linked to Z' and the carrier of a nitrogen which is the carrier of the negative charge.

**4.** The use according to Claims 2 and 3, **characterised by** the fact that when $R_{11}$ represents a negative charge, $R_{10}$ advantageously has at most 30 carbon atoms and is chosen from the hydrocarbonaceous radicals, in particular the aryls, alkyls and silyls, and from the carbonaceous groups attached to Z by a carbon which is both of $sp^2$ hybridization and the carrier of a nitrogen.

**5.** The use according to Claims 2 to 4, **characterised by** the fact that when $R_{11}$ represents a negative charge, $R_{10}$ advantageously has at most 30 carbon atoms and is chosen from the alkyl radicals and silyl and the carbonaceous groups attached to Z by the $sp^2$ hybridization carbon, an iminomethylenyl sequence

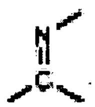

or from a -C O-N< sequence.

**6.** The use according to Claims 4 and 5, **characterised by** the fact that $R_{10}$ has the formula:

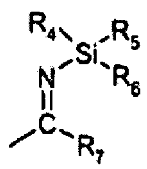

**7.** The use according to Claims 2 to 6, **characterised by** the fact that $R_{10}$ has the formula:

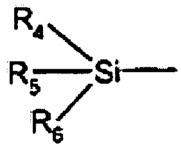

and $R_{11}$ represents a negative charge or has the formula:

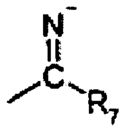

wherein:

. $R_4$, $R_5$ and $R_6$, identical or different, represent a monovalent group, hydrocarbonaceous in nature, which has 1 to 30 carbon atoms, aliphatic, saturated or unsaturated cycloaliphatic, aryl, aralkyl or alkylaryl, possibly substituted by one or more halogen atoms, CN groups, or ester, and/or possibly interrupted by one or more atoms chosen from O, S, N and Si;
. $R_7$ advantageously represents a hydrocarbonaceous radical of at most 30 carbons, advantageously aliphatic in nature (i.e. the carbon of which, the carrier of the link to imine, is of $sp^3$ hybridization), in particular alkyl, or advantageously a preferably isocyclic aryl.

8. The use according to Claims 1 to 7, **characterised by** the fact that the compound with formula (I) corresponds to one of the following formulae (Va) or (Vb):

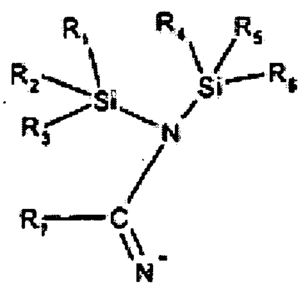

;

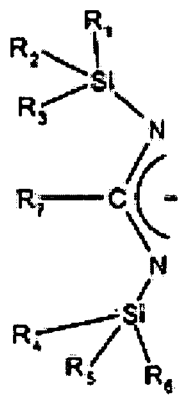

(Va) (Vb)

9. The use according to Claims 1 to 7, **characterised by** the fact that said compound with formula (I) is chosen from the compounds with formulae (1) and (2):

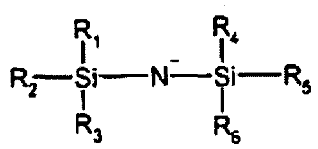

(1)

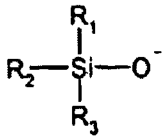 (2)

where $R_1$, $R_2$, $R_3$, $R_4$, $R_5$ and $R_6$, identical or different, represent a monovalent group, hydrocarbonaceous in nature, which has 1 to 30 carbon atoms, aliphatic, satured or unsaturated cycloaliphatic, aryl, aralkyl or alkylaryl, possibly substituted by one or more halogen atoms, CN groups, or ester, and/or possibly interrupted by one or more atoms chosen from O, S, N and Si.

**10.** The use according to Claims 1 to 9, **characterised by** the fact that said cocations are chosen from the oniums and the metal cations, advantageously only having a single stable oxidation state or in their lowest oxidation state (except for the elementary state).

**11.** The use according to Claims 1 to 9, **characterised by** the fact that said cocations are chosen from the alkaline metal cations and the oniums.

**12.** The use according to Claims 1 to 9, **characterised by** the fact that said cocations are chosen from the metal cations of the transition metals.

**13.** The use according to Claims 1 to 10 and 12, **characterised by** the fact that said cocations are chosen from the rare earth cations.

**14.** The use according to Claims 12 and 13, **characterised by** the fact that said cocations are chosen from the lanthanum and lanthanide cations.

**15.** The use according to any of Claims 12 to 14, **characterised by** the fact that said cocations are erbium cations.

**16.** The use according to any of Claims 1 to 10, **characterised by** the fact that said cocations are tin cation (II).

**17.** A (cyclo)condensation process, in particular catalytic (cyclo)trimerisation of monomeric isocyanates, **characterised in that** it comprises:

a) the reaction of the starting monomeric isocyanates with a catalyst comprising a mineral or organic salt of a compound with formula (I);
b) at a temperature of at least 20°C and advantageously of at least 40°C and of at most 200°C, advantageously at most 150°C,
c) stopping the (cyclo)condensation reaction at the desired transformation level, advantageously between 5 and 95%, preferably between 10 and 50%; and
d) possibly eliminating the monomers which have not reacted or the reaction with other compounds reactive with the isocyanate function.

**18.** The (cyclo)condensation process according to Claim 17, in particular catalytic (cyclo)trimerisation of monomeric isocyanates, **characterised by** the fact that the anionic compound with formula (I) is chosen from the compounds corresponding to any of the following formulae:

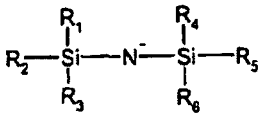 (1)

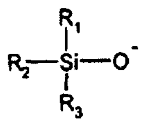

(2)

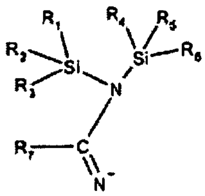

;

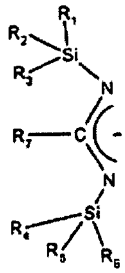

(Va) (Vb)

wherein $R_1$, $R_2$, $R_3$, $R_4$, $R_5$, $R_6$ and $R_7$, identical or different, are as defined in the preceding claims.

**19.** The (cyclo)condensation process according to Claims 17 and 18, in particular catalytic (cyclo)trimerisation of monomeric isocyanates, **characterised by** the fact that the anionic compound with formula (I) is chosen from the compounds below:

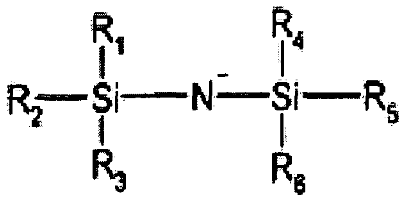

(1)

- with $R_1$, $R_2$, $R_3$, $R_4$, $R_5$ and $R_6$, identical or different, representing a monovalent group, hydrocarbonaceous in nature, having 1 to 30 carbon atoms, aliphatic, saturated or unsaturated cycloaliphatic, aryl, aralkyl or alkylaryl, possibly substituted by one or more halogen atoms, CN groups, or ester, and/or possibly interrupted by one or more atoms chosen from O, S, N and Si,
- one of $R_1$, $R_2$, $R_3$, $R_4$, $R_5$ and $R_6$ represents a pattern with the formula:

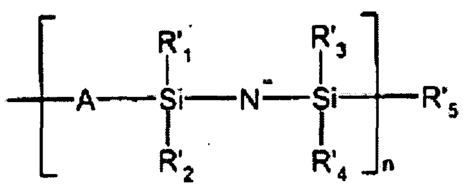

wherein A represents an alkylene chain which has 1 to 30 atoms, advantageously 2 to 20 carbon atoms possibly substituted by one or more halogen atoms, CN groups, or ester, and/or possibly interrupted by one or more atoms chosen from O, S, N and Si, preferably $(CH_2)_{n'}$ with n' being between 1 and 6, advantageously 2 to 6,
and $R'_1$ to $R'_5$, identical or different, representing a monovalent group, hydrocarbonaceous in nature, aliphatic, saturated or unsaturated cycloaliphatic, aryl, aralkyl or alkylaryl, possibly substituted by one or more halogen atoms, CN groups, or ester, and/or possibly interrupted by one or more atoms chosen from O, S, N and Si, and
n is a whole number between 1 and 50, or

- two of $R_1$, $R_2$ and $R_3$ on the one hand and/or $R_4$, $R_5$ and $R_6$ on the other hand, together form a divalent hydrocarbonaceous group, possibly substituted by one or more halogen atoms, CN groups, or ester, and/or possibly interrupted by one or more atoms chosen from O, S, N and Si, and/or

- at least one group chosen from $R_1$, $R_2$ and $R_3$ forms with at least one group of $R_4$, $R_5$ and $R_6$ a divalent hydrocarbonaceous group, possibly substituted by one or more halogen atoms, CN groups, or ester, and/or possibly interrupted by one or more atoms chosen from O, S, N and Si or a precursor of said compound.

**20.** The process according to Claims 17 to 19, **characterised in that** $R_1$ to $R_6$ and $R'_1$ to $R'_5$, identical or different, represent:

- an alkyl, alkenyl, halogenoalkyl or halogenoalkenyl group which has 1 to 20, preferably 1 to 6 carbon atoms, possibly substituted by chlorine and/or fluorine atoms, and/or possibly interrupted by one or more nitrogen and/or silicon atoms,
- a cycloalkyl, cycloalkenyl, halogenocycloalkyl or halogenocycloalkenyl group which has 3 to 30, preferably 3 to 10 carbon atoms and containing chlorine and/or fluorine atoms, and/or possibly interrupted by one or more nitrogen and/or silicon atoms,
- an aryl, alkylaryl or halogenoaryl group which has 6 to 30, preferably 6 to 10 carbon atoms and containing chlorine and/or fluorine atoms,
- a cyanoalkyl group which has 1 to 6 carbon atoms,
- or two groups from $R_1$, $R_2$ and $R_3$ or $R_3$, $R_4$ or $R_5$ together form a divalent radical comprising 2 to 5 carbon atoms, and/or possibly interrupted by one or more nitrogen and/or silicon atoms,
- or two of $R_1$, $R_2$ and $R_3$ on the one hand and/or $R_4$, $R_5$ and $R_6$ on the other hand, together form a divalent hydrocarbonaceous group and/or possibly interrupted by one or more nitrogen and/or silicon atoms, or
- at least one group chosen from $R_1$, $R_2$ and $R_3$ forms with at least one group from $R_4$, $R_5$ and $R_6$ a divalent hydrocarbonaceous group, comprising 2 to 5 carbon atoms, and/or possibly interrupted by one or more nitrogen and/or silicon atoms.

**21.** The process according to Claims 17 to 20, **characterised in that** $R_1$ to $R_6$, identical or different, are chosen from the methyl, ethyl, propyl, isopropyl, butyl, isobutyl, α-pentyl, t-butyl, chloromethyl, dichloromethyl, α-chloro-ethyl, α, β-dichloro-ethyl, fluoromethyl, difluoromethyl, α,β-difluoro-ethyl, trifluoro-3,3,3-propyl, trifluorocyclopropyl, trifluoro-4,4,4-butyl, heptafluoro-3,3,3,4,4,5,5-pentyl, β-cyano-ethyl, γ-cyanopropyl, phenyl, p-chlorophenyl, m-chlorophenyl, dichloro-3,5 phenyl, trichlorophenyl, tetrachlorophenyl, o-, p-, or m-tolyl, α,α,α-trifluorotolyl and xylyls; or two groups from $R_1$, $R_2$ and $R_3$ or $R_4$, $R_5$ and $R_6$ represent a dimethylene, trimethylene, tetramethylene or pentamethylene group and/or $R_1$ and $R_4$ taken together represent a group chosen from ethylene, propylene, butylene, pentylene, preferably non-branched isomers, of methylene and trimethylene.

**22.** The process according to Claims 17 to 21, **characterised in that** $R_1$ to $R_6$ and $R'_1$ $R'_5$, identical or different, are chosen from methyl, ethyl, propyl, vinyl and phenyl, these groups possibly being chlorinated and/or fluorinated.

**23.** The process according to any of Claims 17 to 22, **characterised in that** the compound with formula (1) is chosen from the salts of the following compounds:

hexamethyldisilazane,
diethyl-1,3-tetramethyl-1,1,3,3-disilazane,
divinyl-1,3-tetramethyl-1,1,3,3-disilazane,
hexaethyldisilazane,
diphenyl-1,3-tetramethyl-1,1,3,3-disilazane, hexamethylcyclotrisilazane, and
ethylene-2,5-tetramethyl-2,2,5,5-cyclodisilazane.

**24.** The process according to Claim 18, **characterised in that** the compound with the general formula (2) is chosen from:

trimethylsilanolate,
triethylsilanolate,
ethyldimethylsilanolate, and
vinyldimethylsilanolate.

**25.** The process according to any of Claims 17 to 24, **characterised in that** M represents K, Li, Na or Mg.

**26.** The process according to any of Claims 17 to 24, **characterised in that** the salt of the compound with formula (I) is an erbium salt of the compound with formula (1), and corresponds to the following general formula ($1^E$) when n' represents zero:

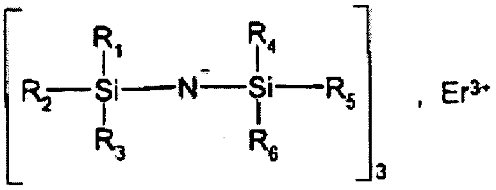

(1^E)

27. The process according to Claim 26, **characterised in that** the erbium salt is chosen from the erbium salts of hexamethyldisilazane, diethyl-1,3-tetramethyl-1,1,3,3-disilazane, divinyl-1,3-tetramethyl-1,1,3,3-disilazane, hexa-ethyldisilazane, and diphenyl-1,3-tetramethyl-1,1,3,3-disilazane.

28. The process according to any of Claims 17 to 24, **characterised in that** the salt of the compound with formula (I) is a tin salt (II) of the compound with formula (1), and corresponds to the following general formula ($1^s$), when n' represents zero:

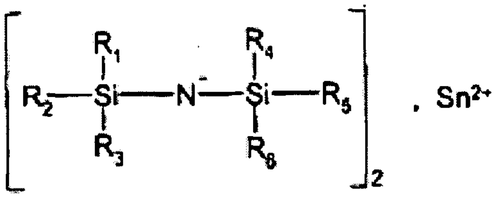

($1^S$)

29. The process according to Claim 28, **characterised in that** the tin salt (II) is chosen from the tin salts of hexamethyldisilazane, diethyl-1,3-tetramethyl-1,1,3,3-disilazane, divinyl-1,3-tetramethyl-1,1,3,3-disilazane, hexa-ethyldisilazane, diphenyl-1,3-tetramethyl-1,1,3,3-disilazane, hexamethylcyclotrisilazane and ethylene-2,5-tetramethyl 2,2,5,5-cyclodisilazane.

30. The process according to any of Claims 17 to 29, **characterised in that** the starting isocyanate is a diisocyanate in which at least one, advantageously the two isocyanate functions are carried by a carbon atom in the cycloaliphatic, secondary, tertiary or neopentylic position.

31. The process according to any of Claims 17 to 30, **characterised in that** said isocyanate is a cycloaliphatic isocyanate wherein at least one, advantageously the two isocyanate functions is/are distanced from the closest cycle by at most one carbon atom, preferably linked directly to the latter.

32. The process according to Claim 31, **characterised in that** the isocyanate is IPDI.

33. The process according to any of Claims 17 to 32, **characterised in that** the molar ratio quantity of catalyst /quantity of NCO functions is at least $5.10^{-5}$, and at most $5.10^{-3}$.

34. An isocyanate composition able to be obtained by the process defined in any of Claims 17 to 33.

35. An isocyanate composition **characterised by** the fact that it comprises compounds which carry isocyanate functions, advantageously aliphatic, and a salt compound chosen from those corresponding to the formula:

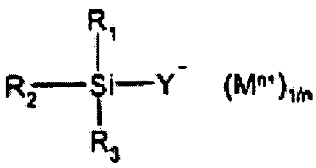

where $(M^{n+})_{1/n}$ is the cocation(s) required for the electric neutrality of said salt compound balancing the anion with

formula (I):

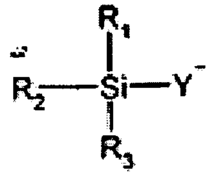

(I)

where Y- is chosen from:

- a negatively charged oxygen;
- or a carbonaceous radical comprising a negative charge carried by an atom from column VB or by an oxygen, advantageously by a nitrogen, and the binding of which with the silicon with the above formula (I) is carried by an atom from column VB, advantageously by a nitrogen;

where $R_1$, $R_2$ and $R_3$, identical or different, represent a monovalent hydrocarbonaceous group advantageously having 1 to 30 carbon atoms, possibly substituted by one or more halogen atoms, CN groups, or ester, and/or possibly interrupted by one or more atoms chosen from O, N and Si, advantageously aliphatic in nature, including saturated or unsaturated cycloaliphatic and aralkyl, or aromatic in nature such as aryl or alkylaryl;
and
from the salts the anionic compound(s) of which (carriers of a negative charge) is/are the carrier/s of a trialkylsilyl group and result(s) from the reaction of said compounds with formula (I) and with an isocyanate function, advantageously aliphatic, the molar ratio quantity of catalyst / quantity of NCO functions being at least $5.10^{-5}$ and at most $5.10^{-2}$.

**36.** The isocyanate composition according to either of Claims 34 and 35, **characterised by** the fact that the concentration of free icosyanate function is between 0.5 and 7 equivalents per litre.

**37.** The isocyanate composition according to any of Claims 34 to 36, **characterised by** the fact that it comprises by mass at least 1%, advantageously at least 3%, preferably at least 5% of compound having at least a pattern:

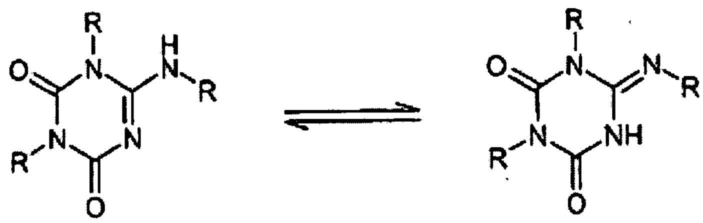

where the Rs, identical or different, represent the remains of the monomer(s), after ignorance of one of the isocyanate functions, the others being able to be engaged in a separate condensation.

**38.** The isocyanate composition according to any of Claims 34 to 36, **characterised by** the fact that it comprises by mass at least 1%, advantageously at least 3%, preferably at least 5% of compound having at least a pattern:

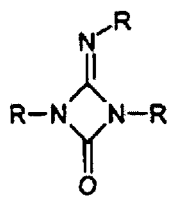

where the Rs, identical or different, represent the remainders of the monomer(s), after ignorance of one of the isocyanate functions, the others being able to be engaged in a separate condensation.

**39.** The isocyanate composition according to any of Claims 34 to 36, **characterised in that** it was implemented with erbium salts, and **characterised in that** it comprises 50 to 80% by weight trimeric compounds consisting of mono-isocyanates and 6-amino-5-aza-uraciles, of 0.5 to 5%, advantageously 0.5 to 3% mono-uretidione compounds, and less than 1% by weight monomeric isocyanates, in relation to the total weight of the isocyanate compounds present in the composition.

**40.** The isocyanate composition according to any of Claims 34 to 36, **characterised in that** it was implemented with tin salts, and **characterised in that** it was implemented with tin salts, and **characterised in that** it comprises 50 to 75%, advantageously 55 to 70% by weight of mono-2-imino-4-oxo-1,3-diazetidine compounds, 10 to 25% by weight, advantageously 12 to 20% by weight of bis-(2-imino-4-oxo-1,3-diazetidine) compounds, 10 to 25%, advantageously 14 to 20% by weight isocyanate ureas, and less than 1 % by weight monomeric isocyanates, in relation to the total weight of the isocyanate compounds present in the composition.

**41.** The isocyanate composition according to any of Claims 34 to 40, **characterised in that** it comprises less than 1% by weight monomeric isocyanates in relation to the total weight of the isocyanate compounds present in the composition, said composition comprising 0.5 to 100 %, advantageously 10 to 100 %, preferably 20 to 100 % masked isocyanate functions with the aid of an isocyanate function masking agent.

**42.** The use of the isocyanate compositions according to any of Claims 34 to 41 in the development of coating products, paints and varnishes, adhesives, inks, biocides, detergents.

## Patentansprüche

**1.** Verwendung einer/von Salzverbindung(en) als Cyclokondensationskatalysator mit vorzugsweise aliphatischer, einschließlich cycloaliphatischer, Isocyanatfunktion, **dadurch gekennzeichnet, dass** die genannte Salzverbindung ausgewählt ist aus denen, die der folgenden Formel entsprechen:

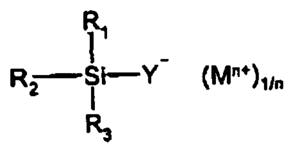

wobei $(M^{n+})_{1/n}$ das/die notwendige(n) Co-Kation(en) für die elektrische Neutralität der genannten Salzverbindung repräsentiert, die das Anion der folgenden Formel (I) ausgleicht:

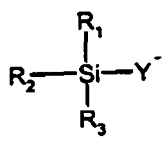

(I)

- wobei $Y^-$ ausgewählt ist aus:

    - einem negativ geladenen Sauerstoff;
    - und einem Kohlenstoffradikal, das eine negative Ladung aufweist, die von einem Atom der Gruppe VB oder von einem Sauerstoff getragen wird, vorzugsweise von einem Stickstoff, und dessen Bindung mit dem Silicium der obigen Formel (I) von einem Atom der Gruppe VB getragen wird, vorzugsweise von einem Stickstoff;

- wobei $R_1$, $R_2$ und $R_3$, die gleich oder unterschiedlich sind, eine einwertige Kohlenwasserstoffgruppe vorteilhafterweise mit 1 bis 30 Kohlenstoffatomen umfasst, optional substituiert durch ein oder mehrere Halogenatome,

CN- oder Estergruppen, und/oder optional unterbrochen durch ein oder mehrere Atome ausgewählt aus O, N und Si, vorteilhafterweise von aliphatischer Natur, einschließlich cycloaliphatischer, gesättigt oder ungesättigt, und aralkylischer Natur, oder von aromatischer Natur wie Aryl oder Aralkyl; und

aus den Salzen, deren anionische Verbindung(en) (Träger einer negativen Ladung) eine Trialkylsilylgruppe tragen und aus der Reaktion der genannten Verbindungen der Formel (I) und einer vorteilhafterweise aliphatischen Isocyanatfunktion resultieren.

**2.** Verwendung nach Anspruch 1, **dadurch gekennzeichnet, dass** Y ein Radikal der Formel II repräsentiert:

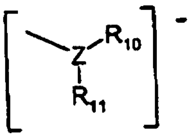

**(II)**

wobei:

- Z ein Metalloid der Gruppe VB repräsentiert;
- $R_{10}$ ausgewählt ist aus Radikalen mit 1 bis 30 Kohlenstoffatomen, einschließlich Silyl und insbesondere Trialkylsilyl; und
- $R_{11}$ ausgewählt ist aus einer negativen Ladung (anionisch) und einer Kohlenstoffgruppe (vorteilhafterweise mit höchstens 30 Kohlenstoffatomen), angelagert an Z über einen Kohlenstoff, gleichzeitig von $sp^{2-}$Hybridisierung und Träger eines Stickstoffs, dessen Stickstoff eine negative Ladung (anionisch) trägt.

**3.** Verwendung nach Anspruch 1, bei der $R_1$, $R_2$ und/oder $R_3$ durch mehrere Atome unterbrochen wird, die ausgewählt sind aus O, N und Si, die die folgende Sequenz (I') bilden:

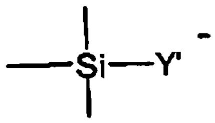

wobei $Y'^{-}$ einen negativ geladenen Sauerstoff oder eine Sequenz (II') repräsentiert:

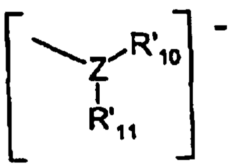

wobei

- Z' ein Metalloid der Gruppe VB repräsentiert;
- $R'_{10}$ eine Kohlenwasserstoffgruppe repräsentiert, die mit einem Z'-Radikal über einen Kohlenstoff oder ein Silicium verbunden ist; (siehe Radikal $R_{10}$);
- $R'_{11}$ die negative Ladung von Y' oder eine Sequenz an einem Stickstoffatom von Y' ist, die einen $sp^2$-Kohlenstoff umfasst, der gleichzeitig an Z' gebunden und Träger eines Stickstoff mit negativer Ladung ist.

**4.** Verwendung nach den Ansprüchen 2 und 3, **dadurch gekennzeichnet, dass**, wenn $R_{11}$ eine negative Ladung repräsentiert, $R_{10}$ vorteilhafterweise höchstens 30 Kohlenstoffatome aufweist und ausgewählt ist aus Kohlenwasserstoffradikalen, insbesondere aus Arylen, Alkylen und Silylen, und aus Kohlenstoffgruppen, die über einen Kohlenstoff an Z angelagert sind, das gleichzeitig von $sp^2$-Hybridisierung und Träger eines Stickstoffs ist.

**5.** Verwendung nach den Ansprüchen 2 bis 4, **dadurch gekennzeichnet, dass**, wenn $R_{11}$ eine negative Ladung repräsentiert, $R_{10}$ vorteilhafterweise höchstens 30 Kohlenstoffatome aufweist und ausgewählt ist aus Alkyl- oder Silylradikalen und Kohlenstoffgruppen, die über den Kohlenstoff mit $sp^2$-Hybridisierung, eine Iminomethylenylsequenz

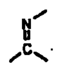

oder eine -C O-N< Sequenz an Z angelagert sind.

**6.** Verwendung nach den Ansprüchen 4 und 5, **dadurch gekennzeichnet, dass** $R_{10}$ die folgende Formel hat:

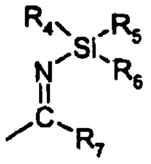

**7.** Verwendung nach den Ansprüchen 2 bis 6, **dadurch gekennzeichnet, dass** $R_{10}$ die folgende Formel hat:

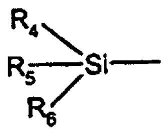

und $R_{11}$ eine negative Ladung repräsentiert oder die folgende Formel hat:

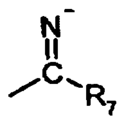

wobei:

- $R_4$, $R_5$ und $R_6$, die gleich oder unterschiedlich sind, eine einwertige Gruppe von Kohlenwasserstoffnatur mit 1 bis 30 Kohlenstoffatomen repräsentiert, aliphatisch, cycloaliphatisch, gesättigt oder ungesättigt, Aryl, Aralkyl oder Alkylaryl, optional substituiert durch ein oder mehrere Halogenatome, CN- oder Estergruppen, und/oder optional unterbrochen durch ein oder mehrere Atome ausgewählt aus O, S, N und Si;
- $R_7$ vorteilhafterweise ein Kohlenwasserstoffradikal mit höchstens 30 Kohlenstoffen repräsentiert, vorteilhafterweise von aliphatischer Natur (d.h. dessen Kohlenstoff, der die Bindung mit dem Imin trägt, $sp^3$-hybridisiert ist), insbesondere Alkyl, oder vorteilhafterweise ein vorzugsweise isozyklisches Aryl.

**8.** Verwendung nach den Ansprüchen 1 bis 7, **dadurch gekennzeichnet, dass** die Verbindung der Formel (I) der folgenden Formel (Va) oder (Vb) entspricht:

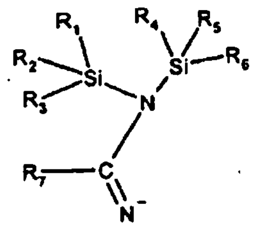

;

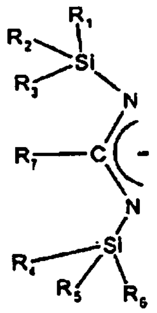

(Va) (Vb)

9. Verwendung nach den Ansprüchen 1 bis 7, **dadurch gekennzeichnet, dass** die genannte Verbindung der Formel (I) ausgewählt ist aus den Verbindungen der Formeln (1) und (2):

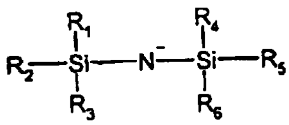

(1)

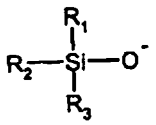

(2)

wobei $R_1$, $R_2$, $R_3$, $R_4$, $R_5$ und $R_6$, die gleich oder unterschiedlich sind, eine einwertige Gruppe von Kohlenwasserstoffnatur mit 1 bis 30 Kohlenstoffatomen sind, aliphatisch, cycloaliphatisch, gesättigt oder ungesättigt, Aryl, Aralkyl oder Alkylaryl, optional substituiert durch ein oder mehrere Halogenatome, CN- oder Estergruppen, und/oder optional unterbrochen durch ein oder mehrere Atome ausgewählt aus O, S, N und Si.

10. Verwendung nach den Ansprüchen 1 bis 9, **dadurch gekennzeichnet, dass** die genannten Co-Kationen ausgewählt sind aus Onium-Ionen und Metallkationen, die vorteilhafterweise nur einen einzigen stabilen Oxidationszustand haben oder in ihrem niedrigsten Oxidationszustand vorliegen (mit Ausnahme des Elementarzustands).

11. Verwendung nach den Ansprüchen 1 bis 9, **dadurch gekennzeichnet, dass** die genannten Co-Kationen ausgewählt sind aus alkalischen Metallkationen und Onium-Ionen.

12. Verwendung nach den Ansprüchen 1 bis 9, **dadurch gekennzeichnet, dass** die genannten Co-Kationen ausgewählt sind aus Metallkationen von Übergangsmetallen.

13. Verwendung nach den Ansprüchen 1 bis 10 und 12, **dadurch gekennzeichnet, dass** die genannten Co-Kationen ausgewählt sind aus den Kationen von Seltenerdmetallen.

14. Verwendung nach den Ansprüchen 12 und 13, **dadurch gekennzeichnet, dass** die genannten Co-Kationen ausgewählt sind aus den Kationen von Lanthan und Lanthaniden.

15. Verwendung nach einem der Ansprüche 12 bis 14, **dadurch gekennzeichnet, dass** die genannten Co-Kationen Erbiumkationen sind.

16. Verwendung nach einem der Ansprüche 1 bis 10, **dadurch gekennzeichnet, dass** die genannten Co-Kationen das Zinn(II)-Kation sind.

**17.** Verfahren zur (Cyclo)kondensation, insbesondere zur katalytischen (Cyclo)trimerisation von Isocyanatmonomeren, **dadurch gekennzeichnet, dass** es beinhaltet:

a) Reagieren der Ausgangsisocyanatmonomere mit einem Katalysator, der ein mineralisches oder organisches Salz einer Verbindung der Formel (I) umfasst;
b) bei einer Temperatur von wenigstens 20°C und vorteilhafterweise von wenigstens 40°C und von höchstens 200°C, vorzugsweise von höchstens 150°C;
c) Stoppen der (Cyclo)kondensationsreaktion bei dem gewünschten Umwandlungsgrad, vorteilhafterweise zwischen 5 und 95 %, vorzugsweise zwischen 10 und 50 %; und
d) optionales Eliminieren von unreagierten Monomeren oder Reagieren mit anderen reaktionsfähigen Verbindungen mit der Isocyanatfunktion.

**18.** (Cyclo)kondensationsverfahren nach Anspruch 17, insbesondere katalytische (Cyclo)trimerisation von Isocyanatmonomeren, **dadurch gekennzeichnet, dass** die anionische Verbindung der Formel (I) ausgewählt ist aus den Verbindungen, die einer der folgenden Formeln entsprechen:

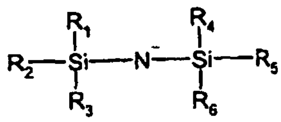
(1)

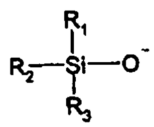
(2)

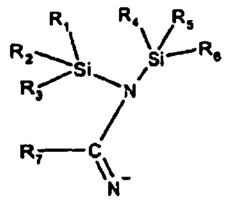 ; 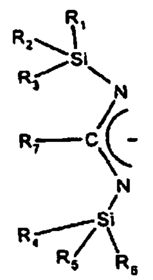

(Va) (Vb)

wobei $R_1$, $R_2$, $R_3$, $R_4$, $R_5$, $R_6$ und $R_7$, die gleich oder unterschiedlich sind, diejenigen sind, die in den vorangegangenen Ansprüchen definiert wurden.

**19.** (Cyclo)kondensationsverfahren nach den Ansprüchen 17 und 18, insbesondere katalytische (Cyclo)trimerisation von Isocyanatmonomeren, **dadurch gekennzeichnet, dass** die anionische Verbindung der Formel (I) ausgewählt ist aus den folgenden Verbindungen:

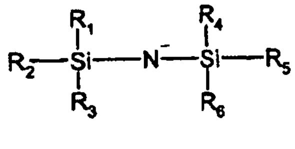
(1)

- wobei $R_1$, $R_2$, $R_3$, $R_4$, $R_5$ und $R_6$, die gleich oder unterschiedlich sind, eine einwertige Gruppe von Kohlenwasserstoffnatur mit 1 bis 30 Kohlenstoffatomen repräsentieren, aliphatisch, cycloaliphatisch, gesättigt oder ungesättigt, Aryl, Aralkyl oder Alkylaryl, optional substituiert durch ein oder mehrere Halogenatome, CN- oder Estergruppen, und/oder optional unterbrochen durch ein oder mehrere Atome ausgewählt aus O, S, N und Si;

- $R_1$, $R_2$, $R_3$, $R_4$, $R_5$ oder $R_6$ eine Einheit der folgenden Formel repräsentiert:

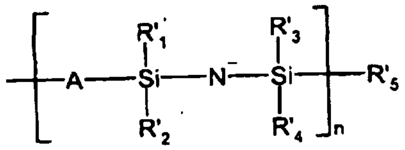

wobei A eine Alkylenkette mit 1 bis 30, vorteilhafterweise mit 2 bis 20 Kohlenstoffatomen umfasst, optional substituiert durch ein oder mehrere Halogenatome, CN- oder Estergruppen, und/oder optional unterbrochen durch ein oder mehrere Atome ausgewählt aus O, S, N und Si, vorzugsweise $(CH_2)_{n'}$, wobei n' zwischen 1 und 6 liegt, vorteilhafterweise 2 bis 6;
und $R'_1$ bis $R'_5$, die gleich oder unterschiedlich sind, eine einwertige Gruppe von Kohlenwasserstoffnatur repräsentieren, aliphatisch, cycloaliphatisch, gesättigt oder ungesättigt, Aryl, Aralkyl oder Alkylaryl, optional substituiert durch ein oder mehrere Halogenatome, CN- oder Estergruppen; und/oder optional unterbrochen durch ein oder mehrere Atome ausgewählt aus O, S, N und Si, und
n eine ganze Zahl zwischen 1 und 50 ist, wobei

- zwei aus $R_1$, $R_2$ und $R_3$ einerseits und/oder $R_4$, $R_5$ und $R_6$ andererseits gemeinsam eine zweiwertige Kohlenwasserstoffgruppe bilden, optional substituiert durch ein oder mehrere Halogenatome, CN- oder Estergruppen, und/oder optional unterbrochen durch ein oder mehrere Atome ausgewählt aus O, S, N und Si, und/oder
- wenigstens eine Gruppe ausgewählt aus $R_1$, $R_2$ und $R_3$ mit wenigstens einer Gruppe aus $R_4$, $R_5$ und $R_6$ eine zweiwertige Kohlenwasserstoffgruppe bildet, optional substituiert durch ein oder mehrere Halogenatome, CN- oder Estergruppen, und/oder optional unterbrochen durch ein oder mehrere Atome ausgewählt aus O, S, N und Si oder einem Vorläufer der genannten Verbindung.

**20.** Verfahren nach den Ansprüchen 17 bis 19, **dadurch gekennzeichnet, dass** $R_1$ bis $R_6$ und $R'_1$ bis $R'_5$, die gleich oder unterschiedlich sind, Folgendes repräsentieren:

- eine Alkyl-, Alkenyl-, Haloalkyl- oder Haloalkenylgruppe mit 1 bis 20, vorzugsweise 1 bis 6 Kohlenstoffatomen, optional substituiert durch Chlor- und/oder Fluoratome und/oder optional unterbrochen durch ein oder mehrere Stickstoff- und/oder Siliciumatome,
- eine Cycloalkyl-, Cycloalkenyl-, Halocycloalkyl- oder Halocycloalkenylgruppe mit 3 bis 30, vorzugsweise 3 bis 10 Kohlenstoffatomen, die Chlor- und/oder Fluoratome enthält, und/oder optional unterbrochen durch ein oder mehrere Stickstoff- und/oder Siliciumatome,
- eine Aryl-, Alkylaryl- oder Haloarylgruppe mit 6 bis 30, vorzugsweise 6 bis 10 Kohlenstoffatomen, die Chlor- und/oder Fluoratome enthält,
- eine Cyanoalkylgruppe mit 1 bis 6 Kohlenstoffatomen,
- oder zwei Gruppen aus $R_1$, $R_2$ und $R_3$ oder $R_3$, $R_4$ und $R_5$, die gemeinsam ein zweiwertiges Radikal mit 2 bis 5 Kohlenstoffatomen bilden, und/oder optional unterbrochen durch ein oder mehrere Stickstoff- und/oder Siliciumatome,
- oder zwei aus $R_1$, $R_2$ und $R_3$ einerseits und/oder $R_4$, $R_5$ und $R_6$ andererseits, die gemeinsam eine zweiwertige Kohlenwasserstoffgruppe bilden und/oder optional durch ein oder mehrere Stickstoff- und/oder Siliciumatome unterbrochen sind, oder
- wenigstens eine Gruppe ausgewählt aus $R_1$, $R_2$ und $R_3$ mit wenigstens einer Gruppe aus $R_4$, $R_5$ und $R_6$ eine zweiwertige Kohlenwasserstoffgruppe bilden, die 2 bis 5 Kohlenstoffatome enthält und/oder optional durch ein oder mehrere Stickstoff- und/oder Siliciumatome unterbrochen ist.

**21.** Verfahren nach den Ansprüchen 17 bis 20, **dadurch gekennzeichnet, dass** $R_1$ bis $R_6$, die gleich oder unterschiedlich sind, ausgewählt sind aus den Gruppen: Methyl, Ethyl, Propyl, Isopropyl, Butyl, Isobutyl, α-Pentyl, t-Butyl, Chlormethyl, Dichlormethyl, α-Chlorethyl, α,β-Dichlorethyl, Fluormethyl, Difluormethyl, α,β-Difluormethyl, Trifluor-3,3,3-propyl, Trifluorcyclopropyl, Trifluor-4,4,4-butyl, Heptafluor-3,3,3,4,4,5,5-pentyl, β-Cyanoethyl, γ-Cyanopropyl, Phenyl, p-Chlorphenyl, m-Chlorphenyl, Dichlor-3,5-phenyl, Trichlorphenyl, Tetrachlorphenyl, o-, p-, oder m-Tolyl, α,α,α-Trifluortolyl und Xylyle;
oder zwei Gruppen aus $R_1$, $R_2$ und $R_3$ oder $R_4$, $R_5$ und $R_6$ eine Dimethylen-, Trimethylen-, Tetramethylen- oder Pentamethylengruppe repräsentieren und/oder $R_1$ und $R_4$ zusammen eine Gruppe repräsentieren, die ausgewählt ist aus Ethylen, Propylen, Butylen, Pentylen, vorzugsweise den unverzweigten Isomeren, von Methylen und Trimethylen.

22. Verfahren nach den Ansprüchen 17 bis 21, **dadurch gekennzeichnet, dass** $R_1$ bis $R_6$ und $R'_1$ $R'_5$, die gleich oder unterschiedlich sind, ausgewählt sind aus Methyl, Ethyl, Propyl, Vinyl und Phenyl, wobei diese Gruppen optional chloriniert und/oder fluoriniert sind.

23. Verfahren nach einem der Ansprüche 17 bis 22, **dadurch gekennzeichnet, dass** die Verbindung der Formel (1) aus den Salzen der folgenden Verbindungen ausgewählt wird:

    Hexamethyldisilazan,
    Diethyl-1,3-tetramethyl-1,1,3,3,-disilazan,
    Divinyl-1,3-tetramethyl-1,1,3,3,-disilazan,
    Hexaethyldisilazan,
    Diphenyl-1,3-tetramethyl-1,1,3,3-disilazan,
    Hexamethylcyclotrisilazan und
    Ethylen-2,5-tetramethyl-2,2,5,5-cyclodisilazan.

24. Verfahren nach Anspruch 18, **dadurch gekennzeichnet, dass** die Verbindung der allgemeinen Formel (2) ausgewählt wird aus:

    Trimethylsilanolat,
    Triethylsilanolat,
    Ethyldimethylsilanolat und
    Vinyldimethylsilanolat.

25. Verfahren nach einem der Ansprüche 17 bis 24, **dadurch gekennzeichnet, dass** M K, Li, Na oder Mg repräsentiert.

26. Verfahren nach einem der Ansprüche 17 bis 24, **dadurch gekennzeichnet, dass** das Salz der Verbindung der Formel (1) ein Erbiumsalz der Verbindung der Formel (1) ist und der folgenden allgemeinen Formel ($1^E$) entspricht, wenn n' nicht null repräsentiert:

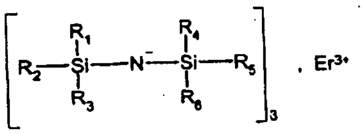

($1^E$)

27. Verfahren nach Anspruch 26, **dadurch gekennzeichnet, dass** das Erbiumsalz ausgewählt wird aus den Erbiumsalzen von Hexamethyldisilazan, Diethyl-1,3-tetramethyl-1,1,3,3-disilazan, Divinyl-1,3-tetramethyl-1,1,3,3-disilazan, Hexaethyldisilazan und Diphenyl-1,3-tetramethyl-1,1,3,3-disilazan.

28. Verfahren nach einem der Ansprüche 17 bis 24, **dadurch gekennzeichnet, dass** das Salz der Verbindung der Formel (1) ein Zinn(II)-Salz der Verbindung der Formel (1) ist und der folgenden allgemeinen Formel ($1^S$) entspricht, wenn n' nicht null repräsentiert:

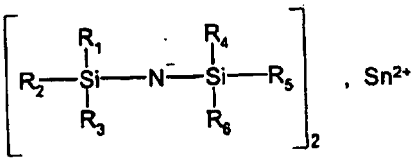

($1^S$)

29. Verfahren nach Anspruch 28, **dadurch gekennzeichnet, dass** das Zinn(II)-Salz ausgewählt wird aus den Zinnsalzen von Hexamethyldisilazan, Diethyl-1,3-tetramethyl-1,1,3,3-disilazan, Divinyl-1,3-tetramethyl-1,1,3,3-disilazan, Hexaethyldisilazan, Diphenyl-1,3-tetramethyl-1,1,3,3-disilazan, Hexamethylcyclotrisilazan und Ethylen-2,5-tetramethyl-2,2,5,5-cyclodisilazan.

**30.** Verfahren nach einem der Ansprüche 17 bis 29, **dadurch gekennzeichnet, dass** das Ausgangsisocyanat ein Diisocyanat ist, in dem wenigstens eine, vorteilhafterweise beide Isocyanatfunktion(en) von einem Kohlenstoffatom in der cycloaliphatischen, sekundären, tertiären oder neopentylischen Position getragen wird.

**31.** Verfahren nach einem der Ansprüche 17 bis 30, **dadurch gekennzeichnet, dass** das genannte Isocyanat ein cycloaliphatisches Isocyanat ist, in dem wenigstens eine, vorteilhafterweise beide Isocyanatfunktion(en) um höchstens ein Kohlenstoffatom, das vorzugsweise direkt damit verbunden ist, vom nächsten Ring beabstandet ist.

**32.** Verfahren nach Anspruch 31, **dadurch gekennzeichnet, dass** das Isocyanat IPDI ist.

**33.** Verfahren nach einem der Ansprüche 17 bis 32, **dadurch gekennzeichnet, dass** das Molverhältnis von Katalysatormenge zur Menge der NCO-Funktionsgruppen wenigstens $5x10^{-5}$ und höchstens $5x10^{-3}$ beträgt.

**34.** Isocyanatzusammensetzung, die mit einem Verfahren erhalten werden kann, das in einem der Ansprüche 17 bis 33 definiert ist.

**35.** Isocyanatzusammensetzung, **dadurch gekennzeichnet, dass** sie Trägerverbindungen von Isocyanatfunktionen, vorteilhafterweise aliphatischen, und eine Salzverbindung umfasst, die ausgewählt ist aus denen, die der folgenden Formel entsprechen:

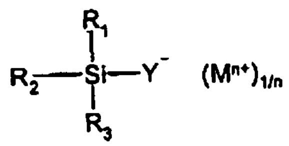

wobei $(M^{n+})_{1/n}$ das/die notwendige (n) Co-Kation(en) für die elektrische Neutralität der genannten Salzverbindung repräsentiert, die das Anion der folgenden Formel (I) ausgleicht:

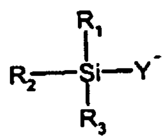

(I)

- wobei $Y^-$ ausgewählt ist aus:

  • einem negativ geladenen Sauerstoff;
  • oder einem Kohlenstoffradikal, das eine negative Ladung aufweist, die von einem Atom der Gruppe VB oder von einem Sauerstoff getragen wird, vorzugsweise von einem Stickstoff, und dessen Bindung mit dem Silicium der obigen Formel (I) von einem Atom der Gruppe VB getragen wird, vorzugsweise von einem Stickstoff;

- wobei $R_1$, $R_2$ und $R_3$, die gleich oder unterschiedlich sind, eine einwertige Kohlenwasserstoffgruppe vorzugsweise mit 1 bis 30 Kohlenstoffatomen umfasst, optional substituiert durch ein oder mehrere Halogenatome, CN- oder Estergruppen, und/oder optional unterbrochen durch ein oder mehrere Atome ausgewählt aus O, N und Si, vorteilhafterweise von aliphatischer, einschließlich cycloaliphatischer Natur, gesättigt oder ungesättigt, und Aralkyl, oder von aromatischer Natur wie Aryl oder Aralkyl; und
aus den Salzen, deren anionische Verbindung(en) (Träger einer negativen Ladung) eine Trialkylsilylgruppe tragen und aus der Reaktion der genannten Verbindungen der Formel (I) und einer vorteilhafterweise aliphatischen Isocyanatfunktion resultieren, wobei das Molverhältnis von Katalysatormenge zur Menge der NCO-Funktionsgruppen wenigstens $5x10^{-5}$ und höchstens $5x10^{-2}$ beträgt.

**36.** Isocyanatzusammensetzung nach einem der Ansprüche 34 und 35, **dadurch gekennzeichnet, dass** die Konzentration an freier Isocyanatfunktion zwischen 0,5 und 7 Äquivalente pro Liter beträgt.

**37.** Isocyanatzusammensetzung nach einem der Ansprüche 34 bis 36, **dadurch gekennzeichnet, dass** sie wenigstens

1 Masse-%, vorteilhafterweise wenigstens 3 Masse-%, vorzugsweise wenigstens 5 Masse-% der Verbindung umfasst, die wenigstens eine Einheit:

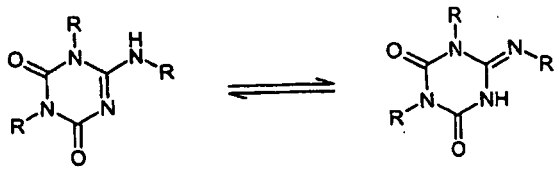

aufweist, wobei die R, die gleich oder unterschiedlich sind, die Reste des oder der Monomers/Monomere repräsentieren, nachdem eine der Isocyanatfunktionen ignoriert wurde, wobei die anderen an einer separaten Kondensation beteiligt sein können.

**38.** Isocyanatzusammensetzung nach einem der Ansprüche 34 bis 36, **dadurch gekennzeichnet, dass** sie wenigstens 1 Masse-%, vorteilhafterweise wenigstens 3 Masse-%, vorzugsweise wenigstens 5 Masse-% der Verbindung umfasst, die wenigstens eine Einheit:

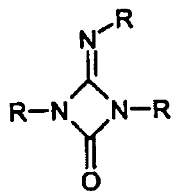

aufweist, wobei die R, die gleich oder unterschiedlich sind, die Reste des oder der Monomers/Monomere repräsentieren, nachdem eine der Isocyanatfunktionen ignoriert wurde, wobei die anderen an einer separaten Kondensation beteiligt sein können.

**39.** Isocyanatzusammensetzung nach einem der Ansprüche 34 bis 36, **dadurch gekennzeichnet, dass** sie mit Erbiumsalzen verwendet werden kann, und **dadurch gekennzeichnet, dass** sie 50 bis 80 Ges.-% an trimeren Verbindungen umfasst, bestehend aus Monoisocyanaten und 6-Amino-5-aza-uracilen, 0,5 bis 5 %, vorteilhafterweise 0,5 bis 3 % Monouretdionverbindungen und weniger als 1 Gew.-% Isocyanatmonomere bezogen auf das Gesamtgewicht der in der Zusammensetzung enthaltenen Isocyanatverbindungen.

**40.** Isocyanatzusammensetzung nach einem der Ansprüche 34 bis 36, **dadurch gekennzeichnet, dass** sie mit Zinnsalzen verwendet werden kann, und **dadurch gekennzeichnet, dass** sie 50 bis 75 %, vorteilhafterweise 55 bis 70 Gew.-% an Mono-2-imino-4-oxo-1,3-diazetidin-Verbindungen, 10 bis 25 Gew.-%, vorteilhafterweise 12 bis 20 Gew.-% an Bis-(2-imino-4-oxo-1,3-diazetidin)-Verbindungen, 10 bis 25 %, vorteilhafterweise 14 bis 20 Gew.-% Isocyanatharnstoffe und weniger als 1 Gew.-% Isocyanatmonomere bezogen auf das Gesamtgewicht der in der Zusammensetzung enthaltenen Isocyanatverbindungen umfasst.

**41.** Isocyanatzusammensetzung nach einem der Ansprüche 34 bis 40, **dadurch gekennzeichnet, dass** sie weniger als 1 Gew.-% Isocyanatmonomere bezogen auf das Gesamtgewicht der in der Zusammensetzung enthaltenen Isocyanatverbindungen umfasst, wobei die genannte Zusammensetzung 0,5 bis 100 %, vorteilhafterweise 10 bis 100 %, vorzugsweise 20 bis 100 % an Isocyanatfunktionen umfasst, die mit Hilfe eines Mittels zum Maskieren der Isocyanatfunktion maskiert sind.

**42.** Verwendung von Isocyanatzusammensetzungen nach einem der Ansprüche 34 bis 41 bei der Herstellung von Beschichtungsprodukten, Farben und Lacken, Klebstoffen, Druckfarben, Bioziden, Detergenzien.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 57653 A


### Littérature non-brevet citée dans la description


- **Houben-Weyl.** Methoden der Organischen Chemie. Georg Thieme Verlag, 1980
- *Organomet. Chem.,* 1983, vol. 461 (1-2), 75-80
- *Chem. Prum.,* 1977, vol. 27 (11), 566-8
- *Zh. Obstich. Khim.,* 1988, vol. 58 (8), 934-5
- *Inorg. Synth.,* 1966, vol. 8, 19-22
- *Inorg. Chem.,* 1966, vol. 8, 15-19
- **Houben Weyl.** Methoden der Organischen Chemie. Georg Thieme Verlag, 1983, 1111-1113
- The determination of ionization constants, a laboratory manual. **A. Albert ; E. P. Serjeant.** The determination of ionization constants, a laboratory manual. Chapman and Hall Ltd
- **Z. Wicks.** *Prog. Org. Chem.,* 1975, vol. 3, 73
- *Prog. Org. Chem.,* 1989, vol. 9, 7
- **Petersen.** *Justus Liebigs, Annalen der Chemie,* 1949, vol. 562, 205